# EUROPEAN PATENT APPLICATION

(11) **EP 3 623 370 A1**
(43) Date of publication of application: **18.03.2020**
(21) Application number: 18382659.3
(22) Date of filing: 13.09.2018
(51) Int. Cl.: C07D 417/12, A61P 35/00, A61K 31/4439, A61K 31/506

(54) **NOVEL TRF1 MODULATORS AND ANALOGUES THEREOF**

(71) Applicant: Fundación del Sector Público Estatal Centro Nacional de Investigaciones Oncológicas Carlos III (F.S.P. CNIO), 28029 Madrid (ES)
(72) Inventor: PASTOR FERNANDEZ, Joaquin Angel, 28029 Madrid (ES); BLASCO MARHUENDA, Maria Antonia, 28029 Madrid (ES); MARTINEZ GONZALEZ, Sonia, 28029 Madrid (ES); BLANCO-APARICIO, Carmen, 28029 Madrid (ES); GARCIA GARCIA, Ana Belen, 28029 Madrid (ES); VARELA BUSTO, Carmen, 28029 Madrid (ES); GOMEZ-CASERO ESTEBAN, Elena, 28029 Madrid (ES); BEJARANO BOSQUE, Leire, 28029 Madrid (ES); MENDEZ PERTUZ, Marinela, 28016 Madrid (ES); MARTINEZ RODRIGUEZ, Paula, 28029 Madrid (ES); GARCIA-BECCARIA, Maria, 28020 Madrid (ES)
(74) Representative: Elzaburu S.L.P.

(57) **Abstract**

Novel TRF1 modulators and analogues thereof.

There is provided compounds of Formula I, wherein R, R1, R2 and X have meanings written in the description. Such compounds are useful as TRF1 inhibitors and, for that reason, as medicaments, in the treatment of cancer, particularly high cancer stem cell cancer like glioblastoma and lung cancer, and can be also useful for the development of additional TRF1 inhibitors and increasing knowledge about TRF1 activity.

## Description

### FIELD OF THE INVENTION

The invention relates to novel compounds that act as TRF1 inhibitors. More particularly, the invention refers to novel compounds that decrease the level of TRF1 proteins in a cell. The invention also relates to the use of such compounds as medicaments, as well as to their use as research tools for the development of additional compounds as drugs against diseases such as cancer, including the research about telomeres and/or TRF1 mechanism of action and activity.

### BACKGROUND OF THE INVENTION

Telomeres are considered potential anticancer targets due to the fact that more than 90% of human tumors aberrantly over-express telomerase (Joseph et al. 2010; Kim et al. 1994; Shay and Bacchetti 1997), while the remaining telomerase-negative tumors activate ALT. In this regard, most studies have focused in telomerase inhibition. The best example are the studies with the telomerase inhibitor GRN163L, also called Imetelstat. However, mouse models of telomerase-based therapeutic strategies have shown some limitations, as the anti-tumorigenic effect is only achieved when telomeres reach a critically short length (Gonzalez-Suarez et al. 2000; Perera et al. 2008) and this effect is lost in the absence of the *p53* tumor suppressor gene, which is commonly mutated in cancer (Chin et al. 1999; Greenberg et al. 1999). In agreement with these findings in mice, human clinical trials with telomerase inhibitors have only shown therapeutic benefits in few myeloid malignancies but have largely failed in solid tumors (Baerlocher et al. 2015; Daniel EI Fassi et al. 2015; Middleton et al. 2014; Parkhurst et al. 2004; Tefferi et al. 2015), maybe as a consequence of telomere length heterogeneity within tumors, which may hamper the effective killing of all tumor cells.

Mammalian telomeres are bound by the so-called shelterin complex formed by the telomere repeat factors 1 and 2 (TRF1 and TRF2), the TRF1-interacting factor 2 (TIN2), the Protection of Telomeres 1 (POT1), the POT1-TIN2 organizing protein TPP1 (also known as TINT1, PTOP or PIP1) and the repressor/activator protein 1 or RAP1 (De Lange 2002, 2005; Liu et al. 2004). TRF1 and TRF2 are bound to double stranded DNA repeats and interact with each other through TIN2 (Houghtaling et al. 2004; Jeffrey Zheng Sheng Ye et al. 2004).The shelterin complex has an indispensable role protecting telomeres from activating DDR and triggering apoptosis and senescence. Interestingly, not only telomerase but also shelterins are often mutated in cancer. Indeed, our group and others have identified POT1 as the first member of telomeric proteins to be mutated in several types of human cancer, both sporadic and familial, including chronic lymphocytic leukaemia (CLL) (Ramsay et al. 2013), familial melanoma (Robles-Espinoza et al. 2014; Shi et al. 2014), Li-Fraumeni like-families (LFL) with cardiac angiosarcomas (CAS) (Calvete et al. 2015), glioma (Bainbridge et al. 2015), mantle cell lymphoma (Zhang et al. 2014), and parathyroid adenoma (Newey et al. 2012).

The fact that shelterins are frequently mutated in cancer supports the notion that targeting shelterins may be a novel and promising strategy to target telomeres in cancer, which would lead to a rapid telomere dysfunction independently of telomere length.

Regarding this matter, previous works of the group of the present inventors have led to the following findings:
1. TRF1 genetic deletion *in vivo* induces a persistent DNA damage response at telomeres, which is sufficient to block cell division and induce senescence and/or apoptosis in different tissues of healthy mice (Martínez et al., 2009)
2. TRF1 is over-expressed in adult stem cell compartments as well as in pluripotent stem cells, where it is essential to maintain tissue homeostasis and pluripotency, respectively (Boué et al. 2010; Schneider et al. 2013)
3. Induction of telomere uncapping by *Trf1* genetic depletion or chemical inhibition can effectively block the growth of rapidly growing lung tumors, in a manner that is independent of telomere length (García-Beccaria et al. 2015). The assays described in said article *Trf1* downregulation by a *Trf1*-shRNA resulted in a markedly delayed tumor onset and growth, while Trf1 chemical inhibition, in turn, effectively impair the growth of already established lung adenocarcinomas without affecting mouse and tissue viability. The authors commented that the results obtained with TRF1 chemical inhibition opened a therapeutic window for targeting TRF1 in cancer that merited further work.
   Chemical inhibition was carried out with two compounds, named ETP-47228 and ETP-47037 in the article, each of them belonging to a different family of compounds: the compounds claimed in international patent applications WO2010119264 and WO2011089400, respectively. The compounds of said families are known inhibitors of the kinase PI3K (phosphatidylinositol 3- kinase).
4. The characterization of the compounds of the families claimed in international applications WO2010119264 and WO2011089400 has led to confirm not only their activity as PI3K inhibitors, but also the ability of PI3K chemical inhibitors, as well as inhibitors of the PI3K downstream target AKT, to reduce TRF1 telomeric foci and lead to increased telomeric DNA damage and fragility, thus uncovering an important functional connection between the PI3K pathway and TRF1 regulation and connecting two of the major pathways in cancer and aging.
5. Subsequent work of the same group (Bejarano *et al.*, 2017) has also demonstrated that TRF1 expression is increased in both human and mouse GBM (glioblastoma multiforme), a deadly and common brain tumor which is considered incurable with the current therapeutic approaches, mainly due to fact that said approaches do not affect glioma stem cells (GSCs), which are able to recapitulate the whole tumor. Bejarano *et al.*, however, have showed that chemical inhibition of TRF1 increases telomeric DNA damage, reduces proliferation and stemness independently of telomere length, thus inhibiting glioblastoma initiation and progression and prolonging survival in genetic and xenograft glioblastoma models, which xenografts models were achieved with human patient-derived primary GSCs. The assays of TRF1 chemical inhibition in glioblastoma models were carried out not only with the PI3K inhibitors named ETP-47228 and ETP-47037 mentioned above, but also with an additional compound, whose structure does not match the structure of the compounds of the families of ETP-47228 and ETP-47037, but that gave rise to similar results when was used as a chemical inhibitor of TRF1, decreasing TRF1 protein levels and inducing telomeric DNA damage and reduced stemness.

The findings described by Bejarano *et al.* in 2017 not only make appear TRF1 as a useful target for cancer treatment in general, but also show that it can be a particularly useful target for cancers with high cancer stem cell content, and very specially in those cases where the current therapies have little effect on cancer stem cells and said cells participate or, even, are crucial for the recurrence of the treated cancer, as happens in glioblastoma. Thus, the search of compounds capable of inhibiting TRF1 has increasing importance as a way of finding new possible drugs for cancer treatment with different properties with regard to solubility, targeted organ, mechanism of TRF1 inhibition, or other properties that can make each compound more appropriate for the treatment of a particular cancer type, a particular way of administration, the avoidance of certain side effects or any other desired properties. Research work directed to increase the knowledge of TRF1 activity and their importance for telomere maintenance (whose impairment is not only involved in cancer, but also in a number of diseases related to ageing (Garcia-Cao et al., 2006)) will also make necessary to have a multiplicity of TRF1 inhibitors, which will allow to select the most appropriate one for each assay.

Thus, there is a need of finding novel TRF1 inhibitors, and also analogues thereof that can be used in related to TRF1 inhibition and their mechanism of action.

The present invention provides a solution to such problem.

### SUMMARY OF THE INVENTION

In accordance to the present invention, there is provided a compound of Formula I, 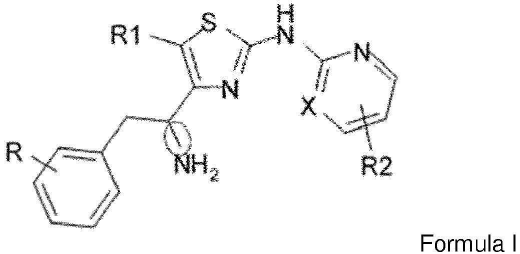 wherein,
R is selected from H, halo, alkyl, O-alkyl (alkyoxy), N-alkyl, alkinyl, O-alkinyl (alkinyloxy),
R1 is selected from H, halo, aryl (unsubstituted or substituted)
X is C or N,
R2 is selected from H, halo, alkyl, O-alkyl, amino, alkenyl, or combinations thereof, and
the bond surrounded by an oval can represent the racemate, the R-enantiomer or the S-enantiomer with regard to the -NH2 group linked by said bond to the structure, provided that
when X is N, R, R1 and R2 are not simultaneously H.

Also an aspect of the present invention is a composition which comprises a compound of the present invention and at least one pharmaceutically acceptable excipient, adjuvant, carrier or diluent.

Another aspect of the present invention is the use of a compound of the invention as a TRF1 inhibitor. The compound can be used for research purposes or for any other purpose where the research of TRF1 is needed.

Yet an aspect of the invention is a compound of the present invention for use as a medicament. The compound can be used, for instance, in the prevention or treatment of cancer, a possible embodiment being that the cancer to treat is one characterized by high cancer stem cells, such as lung cancer or glioblastoma multiforme.

Additionally, it is also an aspect of the invention a composition which comprises a compound of the present invention.

### DESCRIPTION OF THE DRAWINGS

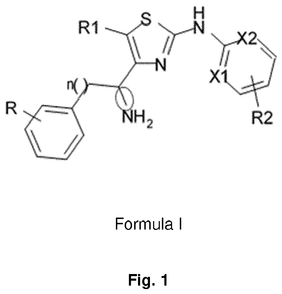

Fig. 1 shows Formula I, the structural formula of the compounds of the present invention.

### DETAILED DESCRIPTION OF THE INVENTION

The present invention stems from the findings recently disclosed by researchers of the group of the present inventors (Bejarano *et al.,* 2017), who reported that targeting telomeres throughout TRF1 inhibition is an effective therapeutic strategy of GBM, since TRF1 inhibition (and, particularly, TRF1 protein decrease) was found to impair GBM initiation and progression and to prolong survival in genetic and xenograft glioblastoma models, through a mechanism that involves telomeric DNA damage induction and reduced stemness. Among the compounds that were shown to act as TRF1 inhibitors and to give rise to the mentioned effects is a compound that Bejarano *et al.* reported to by represented by the following formula:

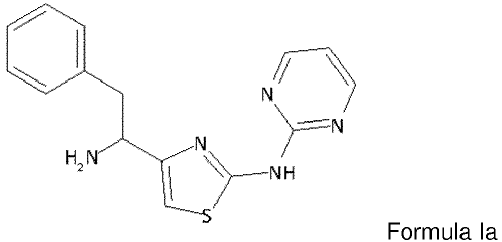

The compound represented by Formula Ia is a racemic compound included into a Kinase Inhibitor Library sourced from BioFocus (Galapagos, Belgium). As its structure does not match the structures of the families of PI3K inhibitors to which belong the other compounds used as TRF1 inhibitors in the same article (Bejarano et al., 2017), the present inventors decided to broaden the study of its properties and the properties of their enantiomers, the compounds ETP-50946 and ETP-51905, which are depicted below.

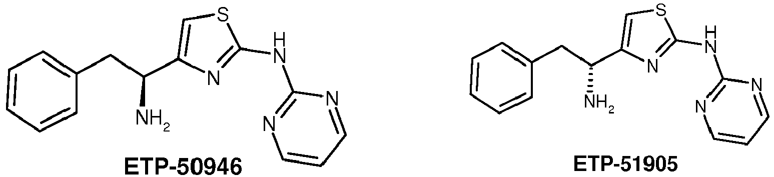

The inventors decided to select the compound of Formula Ia and their enantiomers as hit compounds to start to development of novel TRF1 modulators, by carrying out different modifications in different positions in the chemical structure of the initial hits, trying to find modifications which do not compromise the anti-TRF1 activity of these analogues. For that reason, and as used herein, the term "S-enantiomer" (S), "R-enantiomer" (R) and racemate or racemic mixture (rac), when used with regard to the compounds of the present invention, always refer to stereoisomery with regard to the - NH2 group present in the compound of Formula Ia and/or their enantiomers.

It has been found that only some parts of the molecule allow the introduction of modifications to maintain TRF1 inhibition, other ones being detrimental. Surprisingly, some of the modifications occurred to be detrimental only when they derive from a particular enantiomer (ETP-50946 or ETP-51905) and not from the other one, while other modifications have the same effect regardless of the starting enantiomer. Given the strong structural similarities among some of the novel compounds that show TRF1 inhibitor activity and those that show no activity, some of the novel compounds that show no activity have been also considered part of the present invention, due to their possible utility as controls in future research assays related to the mechanism of action and physical interactions of these family of compounds.

In particular:
R can be: H, halo (fluoro, bromo, chloro), alkyl (preferably, C₁₋₁₂-alkyl), -Oalkyl (preferably, C₁₋₁₂-alkyloxy, such as methyloxy:-OCH₃), NH₂, N-alkyl, alkinyl, O-alkinyl (alkinyloxy). Preferably, it is: H, halo (fluoro, chloro, bromo) or O-alkinyl.

With regard to R, the substituent of the phenyl group, it has been observed that *ortho* (o-), *meta (m-)* and *para (p-)* substitutions with an halo substituent (F, Cl, Br), give rise to active compounds, regardless of the stereoisomery with regard to the NH2 group. The effect of the substitution with alkinyloxy groups (-O(CH₂)ₘ(CCH)) varied depending on the value of m (1, 2, 3, 4...), the substituted position in the phenyl group and the stereoisomery with regard to the NH2 group: *meta* substitution with propinyloxy (-OCH₂CCH) give rise to active compounds regardless of the stereoisomery with regard to the NH₂ group (see compounds 17, 18, 19), whereas para-substitutions with the same substituent result in an inactive R-enantiomer (see compound 22) but active racemate and S-enantiomer (see compounds 20 and 21). Analogous results were found with the para-hexinyloxy (-O(CH₂)₄CCH) where, curiously, only the S-enantiomer was active (see compounds 26, 27 and 28). As it is explained below, given its potential future applicability as research tools, all compounds with an alkinyloxy group as R substituent have been considered compounds of the present invention. Substitution with -NO₂, in turn, was not found active and the compounds where R is -NO₂ are not compounds of the present invention. Consequenty:
- When R is halo (fluoro, chloro or bromo), the R substituent can be in *ortho* (o-), *meta (m-)* or *para (p-)* position with regard to the C atom by which the phenyl group is linked to the rest of the molecule, and the amino group of Formula I can be attached so that the compound is any one of the enantiomers (the S-enantiomer or the R-enantiomer) or the racemate (racemic mixture: rac).
- When R is -Oalkinyl, it is preferably selected from the group of -O(CH₂)ₘ(CCH), wherein m is 1, 2, 3 or 4. The R substituent can be in *ortho* (o-), *meta (m-)* or *para (p-)* position with regard to the C atom by which the phenyl group is linked to the rest of the molecule. *Meta* and *para* substitutions are preferred. When the substituent
- O(CH₂)ₘ(CCH) is in the *para* position, there are differences in the activity of the racemate and different enantiomers with regard to the amino group of Formula I.
- When R is alkinyl (such as, for instance, -CCH), the substituent can be in *ortho* (o-), *meta (m-)* or *para (p-)* position with regard to the C atom by which the phenyl group is linked to the rest of the molecule. When R is in *meta* position, the activity is similar for the two enantiomers or the racemate; when R is in *para* position, one of the enantiomers shows higher activity than the other enantiomer and the racemate.

X can be N or C. When X is N, the ring is a pyrimidinyl ring, attached to the rest of the molecule by position 2 of the ring (2-pyrimidinyl), as in the starting compounds. But it is also possible that X is C, so that the ring can be a 2-pyridyl ring. The pyrimidinyl and the pyridyl ring can be unsubstituted (R2 = H) or substituted.

When the ring is a pyrimidinyl ring (X is N), both *meta* (position 4 of the ring) and *para* (position 5 of the ring) are possible for the R2 substituent. It is even possible to have two R2 substituents, each one different from the other (for instance, CH₃ and -OCH₃).

R2 (the possible substituent of the ring of X) is selected from H, halo (fluoro, chloro, bromo), alkyl (for instance, C₁₋₁₂ alkyl), alkyloxy (where, for instance, the alkyl moiety is a C₁₋₁₂ alkyl), amino, alkenyl, or combinations thereof. For instance, in compound 37, there is a different substituent in each one of the *meta* positions: an alkyl (CH₃) and an -Oalkyl (methyloxy: OCH₃), being an active compound, although its activity is slightly decreased with regard to other compounds such as, for instance, compound 33, where only one R2 substituent, namely CH3, is present in the *meta* position (see Table 1). Preferred embodiments are those where R2 is H, bromo, CH₃ or CCH.

R1 can be H, halo or substituted or unsubstituted aryl. It is preferred that it is selected from H, bromo or substituted phenyl (Ph), where the substituent of the phenyl can have an alkenyl or Oalkinyl moiety at one end (that is, R1 can be Ph-(O)ₙ₁(CH₂)ₙ₂(CCH), where n1 can be 0 or 1, and n2 can be, preferably, 0, 1, 2, 3, 4), like, for instance, PhCCH or Ph-O-CH₂CCH. The presence of the alkinyl will allow the use of the compound as research tool linked to click chemistry (see below). Also active are the compounds where R2 is PhNH₂, Ph-NHCOCH₃, PhOH, Ph-O-CH₂CH₂OCH₃.

It is important to note that TRF1 inhibition is measured at cellular level and the permeability of the different analogues can influence their values and the interpretation of the SAR results. This is also a possible line of research where the family of compounds of the present invention might be of help to associate different structural modifications with variations in permeability that might also help to develop TRF1 inhibitors well adapted to the tissue where they should exert their action, and/or to the administration route or even to a particular vehicle from which they might be released. And, given that TRF1 is part of the shelterin complex that protects telomeres, the compounds might also be useful in further research about telomeres and disorders related to telomere length and ageing, such as age-related disorder is selected from the group of osteoporosis, arthrosis, glucose intolerance, insulin resistant, reduced heart, circulatory and/or lung function, cardiovascular disease, loss of memory, loss of neuromuscular coordination and decrease of longevity, or combinations thereof.

Among the compounds of the present invention, it is worth mentioning those compounds where R is an alkynyl group or which present an alkynyl moiety at the end of the R, R1 or R2 substituent, because such compounds could be particularly useful as research tools, particularly as biomolecule labeling tools (nucleic acid labeling tools or, particularly in the present case, protein labelling tools) linked to the so-called "click chemistry"; a reaction between azide and alkyne yielding as covalent product 1,5-disubstituted 1,2,3.triazole. Due to the most common conditions of the reaction, the process is also known as CuAAC: catalyzed alkyne azide cycloaddition, although other preparation process are also possible (see, for instance, the review of Singh et al., 2016). Click chemistry has found applications in very different areas, including bioconjugation, drug discovery, materials science and radiochemistry (see, for instance, the revision of New and Brechbiel, 2009), so that the compounds of the invention where one of the R, R1 or R2 subtituents is an alkynyl group or ends with an alkynyl moiety can be particularly useful as research tools in different fields, remarkably as precursors to make chemical proofs for cell imaging studies and target deconvolution experiments including cellular localization studies and pull down experiments. Examples of such compounds are the compounds where:
R is selected from: -CCH, -OCH₂CCH and -O(CH₂)₄CCH, or
R1 is selected from: Phenyl-CCH, Phenyl-O-CH₂CCH, or
R2 is selected from : R2 is 5-CCH, 5-NH-CO-(CH₂)₂CCH

The novel synthesized compounds, the methods used to synthesize them and their activities are described in detail below.

### Medical and Pharmaceutical Uses

As indicated above, the TRF1 inhibition activities of most compounds of the present invention lead to the possibility of using any of such compounds as medicaments. Thus, the compounds with TRF1 inhibition activity may be of use in the treatment of prevention of a disease or disorder in which the inhibition of TRF1 activity is desired or required. Thus, it is possible to conceive as aspects of the present invention both a compound of the present invention for use as a medicament and a method for treating a disease or disorder linked to excessive TRF1 activity by the administration of a compound of the present invention to a subject which in need therefore. It can be also considered an aspect of the present invention a pharmaceutical composition or a composition which comprises a compound of the present invention and at least one pharmaceutically acceptable excipient, adjuvant, carrier or diluent.

As it has been commented above, previous works of the group of the present inventors (García-Beccaria et al., 2015; Bejarano et al., 2017) have shown the therapeutic potential of TRF1 inhibition for cancer treatment and prevention, specially in the case of aggressive and difficult to treat tumors such as lung carcinoma (Garcia-Baccaria et al., 2015) or glioblastoma multiforma (GBM) (Bejarano et al., 2017). Both of them are tumour with high cancer stem cell involvement, which cells, and very particularly in the case of GBM, are not affected by the current therapies, make difficult the treatment and are responsible for the recurrence. The compounds of the present invention, which derive from the TRF1 inhibitor of Formula Ia whose effectiveness in glioblastoma models has been reported by Bejarano et al. (2017), may be useful in the treatment or prevention of cancer and, very specially, in the treatment or prevention of a high cancer stem cell content cancer such as lung carcinoma or glioblastoma multiforme.

Although some compounds of the invention may possess pharmacological activity as such, it might also happen that they are not administered to the subject in need of them as such compound, but as a prodrug, that is, a compound that is metabolized in the body and give rise to the compound with pharmaceutical, veterinary or medical activity. The medical use of prodrugs of the compounds of the invention is also comprised within the scope of the present invention.

The compounds could be administered in the form of a composition. Thus, the compositions of the active compounds of the present invention, and preferably those ones which also comprise a pharmaceutically acceptable excipient, diluent or carrier, are also compositions of the present invention. Given the difficulties associated to cancer treatment, the composition can additionally comprise another antitumoral compound, which can be least an additional TRF1 inhibitor. As commented before, previous works of the present inventors have shown that compounds that act through the Akt/PI3K pathway (such as the compounds claimed in international patent applications WO2010119264 and WO2011089400) (Mendez-pertuz et al., 2017) have activity as TRF1 inhibitors, so that the additional TRF1 inhibitor can be one of such compounds. It is also possible that the compound is the starting compound for the compounds of the present invention (the compound of Formula I where X is N, and R, R1 and R2 are H), which has been shown to have TRF1 inhibition activity (Bejarano et al., 2017), so that it can be one of the selected compounds for the composition. Additionally, it is possible to use a compound selected from the group of: an RTK inhibitor, a MEK inhibitor, an ERK inhibitor, an HSP90 inhibitor, docetaxel and gemcitabine, which have been also shown to have TRF1 inhibition activity and, besides, show a synergistic effect with TRF1 inhibitors, particularly those that act through the Akt/PI3K pathway. But the compound can also be another compound with known activity as antitumoral and not previously linked to TRF1 inhibition activity, such as temozolomide, a compound used in the treatment of glioblastoma.

As the compounds of the present invention, all the above mentioned compositions can be for use as a medicament, particularly for use in the prevention or treatment or cancer, which cancer can be one characterized by high content of cancer stem cells, such as glioblastoma or lung carcinoma.

The combination of the administration of any of the compounds of the present invention with radiotherapy is also advisable, and it is also part of the present invention, as a method of treatment, particularly recommended for subjects (mammals, particularly human beings) in need thereof, specially if they suffer from glioblastoma, because the group of the present inventors have previously shown that TRF1 inhibitors have a synergistic effect with radiotherapy in glioblastoma.

As used herein, TRF1 inhibition refer to any measurable reduction or decrease of the activity of TRF1 protein. Such decrease of activity may result from a decrease of the expression of the gene, from a decrease in protein levels that may be or not the result of a decrease in the expression of the gene, or from any condition in a cell or reaction mixture that give rise to a decrease of the activity of the protein, even though the protein levels may remain to be the same; the latter situation might result, for instance, from the presence of a compound that interacts directly with TRF1 protein and hinders its activity. As a download in the expression of the gene might be the result of the modulation of the activity of another gene or protein that is involved in the regulation of TRF1 expression, the term "inhibitor" does not only comprise those compounds which interact directly with TRF1 protein or its gene, but also those compounds that give rise to a decrease in TRF1 protein activity by interacting directly with another gene or protein or having an influence in the expression of such gene.

In the case of TRF1, its binding to telomeres can be considered its activity. For that reason, the present inventors have measured TRF1 levels at telomere by immunofluoresce, as detailed below. Thus, as it is used herein, those compounds of the present invention that gave rise to a decrease in TRF1 levels at telomeres were considered TRF1 inhibitors or compounds with TRF1 activity, thus being candidates for being used as drugs in medicaments of for the use in the treatment or prevention of a disease related to TRF1 activity, such as cancer. Table 2, in Example 2, show the activity of some compounds of the present invention, measured as commented above. It is comprised within the scope of the present invention any of such the compounds of Table 2 whose activity is higher than 0 for use as a medicament and, more particularly, for use in the treatment of cancer, specially a high cancer stem cell tumor such as glioblastoma or lung carcinoma.

### EXAMPLES AND EXPERIMENTAL

The following Examples illustrate the invention.

### List of abbreviations:

Hereinafter, the term, "DCM" means dichloromethane, "DIPEA" means diisopropylethylamine, "TEA" means triethylamine, "DMAP" means dimethylaminopyridine, "DME" means 1,2-dimethoxyethane, "DMF" means dimethylformamide, "TFA" means trifluoroacetic acid, "eq" means equivalents, "EtOAc" means ethyl acetate, "h" means hours, "min" means minutes, "HPLC" means high performance liquid chromatography, "MeOH" means methanol, "mw" means microwave, "nBuOH" means n-butanol, "NMR" means nuclear magnetic resonance, "Pd(PPh₃)₄" means tetrakis(triphenylphosphine)-palladium, "THF" means tetrahydrofuran, "CHCl₃" means chloroform, "PdCl₂dppf" means 1,1'-bis(diphenylphosphino)ferrocene-palladium(II)dichloride dichloromethane complex, "AcCN" means acetonitrile, "Na₂SO₄" means sodium sulphate, "rt" means room temperature, "c-Hex" means cyclohexane, "CDCl₃" means deuterated chloroform, "DMSO" means dimethylsulfoxide, "NaHCO₃" means sodium bicarbonate, "H₂O" means water, "NaCl" means sodium chloride, "NH₄Cl" means ammonium chloride, "Na₂S₂O₃" means sodium thiosulfate, "EtOH" means ethanol, "AcOH" means acetic acid, "KOH" means potassium hydroxide, "Na₂CO₃" means sodium carbonate, "aq" means aqueous, "EDA" means etrhylendiamine.

### General Procedure for characterization of compounds:

NMR spectra were recorded in a Bruker Avance II 300 spectrometer and Bruker Avance II 700 spectrometer fitted with 5 mm QXI 700 S4 inverse phase, Z-gradient unit and variable temperature controller.

The HPLC measurements were performed using a HP 1100 from Agilent Technologies comprising a pump (binary) with degasser, an autosampler, a column oven, a diode-array detector (DAD) and a column as specified in the respective methods below. Flow from the column was split to a MS spectrometer. The MS detector was configured with an electrospray ionization source or API/APCI. Nitrogen was used as the nebulizer gas. Data acquisition was performed with ChemStation LC/MSD quad, software.

### Method 1

Reversed phase HPLC was carried out on a Gemini-NX C18 (100 x 2.0 mm; 5 um). Solvent A: water with 0.1% formic acid; Solvent B: acetonitrile with 0.1% formic acid. Gradient: 5% to 100% of B within 8 min at 50 ºC, DAD.

### Method 2

Reversed phase HPLC was carried out on a Gemini-NX C18 (100 x 2.0 mm; 5 um).

Solvent A: water with 0.1% formic acid; Solvent B: acetonitrile with 0.1% formic acid. Gradient: 5% to 40% of B within 8 min at 50 ºC, DAD.

### Method 3

Reversed phase HPLC was carried out on a Gemini-NX C18 (100 x 2.0 mm; 5 um). Solvent A: water with 0.1% formic acid; Solvent B: acetonitrile with 0.1% formic acid. Gradient: 0% to 30% of B within 8 min at 50 ºC, DAD.

### Method 4

Reversed phase HPLC was carried out on a Gemini C18 column (50 x 2 mm, 3 um). Solvent A: water with 0.1% formic acid; Solvent B: acetonitrile with 0.1% formic acid. Gradient: 10% to 95% of B within 4 min at 50ºC, DAD.

### Method 5

Reversed phase HPLC was carried out on a Gemini C18 column (50 x 2 mm, 3 um). Solvent A: water with 0.1% formic acid; Solvent B: acetonitrile with 0.1% formic acid. Gradient: 0% to 30% of B within 4 min at 50ºC, DAD.

"Found mass" refers to the most abundant isotope detected in the HPLC-MS. Rt means Retention time in min and [M+H]+ is the corresponding mass of the compound + 1.

### - Example 1: Synthesis of intermediates and final products

### General Methods

### -Method A: Protection of amino with phtalamide

A mixture of the corresponding amino precursor (1 eq) and phthalic anhydride (1 eq) in acetonitrile was heated in a sealed tube at 145 ºC till completion of the reaction. On cooling, the mixture was evaporated to give the expected product.

### -Method B: Acyl chloride formation

To a suspension of the corresponding carboxylic acid precursor (1 eq) in toluene was added SOCl₂ (15 eq). The mixture was refluxed (125 ºC) for 1 h. On cooling, the mixture was concentrated and the residue was co-evaporated with toluene (x3) to give the expected product which was used in the next step with no further treatment.

### -Method C: Synthesis of αBromo ketones from acyl chloride

To a solution of corresponding acyl chloride precursor (1 eq) in THF was added dropwise a solution of (trimethylsilyl)diazomethane (2M in hex, 2.5 eq) at 0 ºC. The reaction mixture was stirred at 0 ºC for 30 min and at rt for 1 h. The yellow solution was concentrated. The residue was dissolved in AcOH (30 mL) and HBr 48% aq (2.5 eq) was added at 0 ºC. The reaction mixture was stirred at 0 ºC -10 ºC for ca. 20 min and concentrated. The residue was taken in EtOAc and washed with sat. NaHCO₃. The organic layer was dried, filtered and evaporated to give the expected product which was used in the next experiments with no further treatment.

### -Method D: Thiazole formation

To a solution of the corresponding bromo ketone precursor (1.0 eq) in DMF was added the corresponding R1-thiourea (1.0 eq.) and stirred at room temperature overnight. Water was added, and the suspension was filtered, washing with water. The solid obtained was dried in a vacuum oven to yield the pure expected product.

### -Method E: Phtalamide deprotection

A mixture of the corresponding precursor (1 eq) and hydrazine hydrate (15 eq) or methyl hydrazine hydrate (15 eq) in EtOH was heated in a sealed tube at 80 ºC for 16 h. On cooling, the mixture was concentrated and the residue was purified by chromatography in silica to give the expected product.

### -Method F: Chiral Separation

Racemate mixtures were subjected to chiral separation by preparative HPLC using a chiral column chromatography to give the corresponding enantiomers with unknown configuration. The configuration of the compounds is randomly assigned: First elution peak is drawn with up wedge bond and the second elution peak is drawn with down wedge bond. In order to facilitate the references and denominations of each enantiomer, the compound which corresponds to the first elution peak (up wedge bond in the structural formulae) has been named the S-enantiomer, while the compound which corresponds to the second elution peak (down wedge bond in the structural formulae) has been named the R-enantiomer.

### -Method G: Ester formation

A mixture of the corresponding acid precursor (1 eq) in MeOH (12 mL) was added SOCl₂ (1.3 eq) dropwise at 0 ºC. The resulting solution was heated at 75 ºC for 21 h. On cooling, the mixture was concentrated to give the expected product.

### -Method H: BOC Amine protection

A solution of the corresponding amino precursor (1 eq) in dioxane:H₂O (1:1) was added NaHCO₃ (3 eq) and Boc₂O (1.2 eq). The reaction mixture was stirred at rt for 2h. Dioxane was removed in vacuum, EtOAc was added and the mixture was washed with H₂O and brine. The organic layer was dried, filtered and evaporated to give the expected product.

### -Method I: O-Alkylation

A mixture of the corresponding precursor (1 eq) and alkylating agent (1.5 eq) and K₂CO₃ (2.5 eq) in DMF was stirred at rt till completion of the reaction. H₂O was added and the mixture was extracted with EtOAc. The organic layer was dried, filtered and evaporated. The crude compound was used in next reaction step without further purification (it can be impure with the corresponding precursor).

### -Method J: BOC amino deprotection

A solution of the corresponding precursor BOC protected amino derivative (1 eq) in 4N HCI in dioxane was stirred at rt for 15 h. The mixture was concentrated to give the expected product as HCI salt.

### -Method K: Ester Hydrolisis

To a solution of the corresponding ester precursor (1 eq) in MeOH was added a solution of NaOH (3 eq) in H₂O. The reaction mixture was stirred at rt till completion of the reaction. H₂O was added and the pH was adjusted to 3-4 with cHCl. The mixture was concentrated to give the expected product and it was used in the next experiment with no further treatment.

### -Method L: Halogenation

To a suspension of the corresponding precursor (1 eq) in DMF or AcCN was added NIS (1.1 eq) or NBS (1.1 eq). The reaction mixture was stirred at rt till completion of the reaction. H₂O was added and the mixture was filtered and rinsed with H₂O to give the expected product. The compound was purified by chromatography in silica gel if necessary.

### -Method M: Suzuki coupling

A mixture of the corresponding halo-derivative precursor (1 eq), the corresponding boronic or boronate ester reagent (1.5 eq),), PdCl₂(PPh₃)₂ (0.1 eq) and Na₂CO₃ 2M (3 eq) in dioxane was heated in sealed tube at 100 ºC till completion of the reaction. On cooling, the mixture was concentrated and the residue was purified by chromatography in silica gel to give the expected product.

### -Method N: Boc Amino deprotection

To the corresponding BOC-amino derivative precursor (1 eq) in was added TFA (20 equiv). The mixture was stirred at rt overnight and then concentrated and the residue was purified by SCX-2 column to render expected product as free base which was triturated with acetonitrile to yield final compound pure.

### -Method R: Sonogashira reaction

The corresponding halo aromatic derivative (1 equiv), trimethylsylylacetylene (3 equiv), PdCl₂(PPh₃)₂ (1.0 equiv), CuI (1.0 equiv), Et₃N (7.0 equiv) were stirred in DMF at 120 ºC till completion of the reaction. The solvent was concentrated and the reaction mixture was purified by chromatography in silica gel.

### -Method S: Amino Boc or TMS deprotection with Amberlyst®

To a solution of the corresponding precursor (1 eq) in MeOH was added Amberlyst(r) 15 (∼15 eq). The reaction mixture was stirred at rt till no starting material is detected by TLC and filtered. The resine was suspended in 7N NH₃-MeOH, stirred for 1 h and filtered. This treatment was repeated twice. The filtrates were evaporated and the residue was purified by chromatography in silica gel to give the expected product deprotected.

### -Method T: Amide formation

The corresponding amino derivative precursor (1 equiv) in DMF was treated with DIPEA (5 eq) and the corresponding carboxylic acid (2 equiv) in DMF. Then HOBt (2 equiv), HATU (2 equiv) were added, stirring the reaction mixture at room temperature till completion of the reaction. Water was added and the reaction was extracted with EtOAc. Organic layer was separated, dried (Na₂SO₄), filtered and evaporated to dryness. The residue was purified by chromatography in silica gel to yield the expected products.

### Synthesis of Intermediates

### Synthesis of Intermediate I

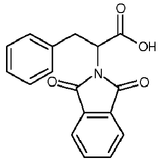

Intermediate I was prepared following Method A from DL-phenylalanine (1 g, 1 eq) and phthalic anhydride (900 mg, 1 eq) in acetonitrile (25 mL) to yield 1.73 g, 96%, beige solid. 1H NMR (700 MHz, DMSO) δ 7.90 - 7.78 (m, 4H), 7.23 - 7.08 (m, 5H), 5.12 (dd, *J* = 11.7, 4.8 Hz, 1H), 3.51 (dd, *J* = 14.2, 4.8 Hz, 1H), 3.38 (dd, *J* = 14.1, 11.8 Hz, 1H).

### Synthesis of Intermediate II

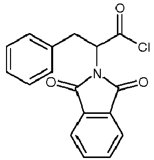

Intermediate II was prepared following Method B from Intermediate I (1.73 g, 1 eq) in toluene (60 mL) at reflux, to yield the expected product (quantitative yield assumed).

### Synthesis of Intermediate III

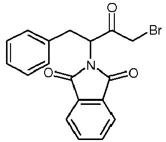

Intermediate III was prepared following Method C from Intermediate II (1.85 g, 1 eq) in THF (40 mL); AcOH (30 mL) to yield 2.16 g, 98%, brown oily-solid (TLC: 30% EtOAc in cHex).

### Synthesis of Intermediate IV

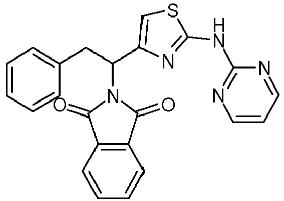

Intermediate IV was prepared following Method D from Intermediate III (6.41 g, 1 eq) and 1-(pyrimidin-2-yl)thiourea (2.66 g, 1 eq) in DMF (86 mL) to yield 6.33 g, 86%, beige solid. HPLC-MS (method 4): Rt = 4.5 min [M+H]+ 428.2.
1H NMR (300 MHz, DMSO) δ 11.66 (s, 1H), 8.61 (d, *J* = 4.8 Hz, 2H), 7.81 (d, *J* = 7.8 Hz, 4H), 7.28 - 7.14 (m, 5H), 7.01 (t, *J* = 4.9 Hz, 1H), 5.58 (dd, *J* = 10.1, 6.5 Hz, 1H), 3.68 - 3.60 (m, 2H).

### Synthesis of Intermediate V

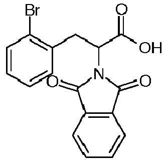

Intermediate V was prepared following Method A from 2-bromo-DL-phenylalanine (750 mg, 1 eq) and phthalic anhydride (455 mg, 1 eq) in acetonitrile (8 mL) to yield 1.1 g, 90%, beige solid.
¹H NMR (300 MHz, DMSO) δ 7.84 (m, 4H), 7.56 - 7.47 (m, 1H), 7.16 - 7.02 (m, 3H), 5.13 (dd, *J* = 11.5, 4.2 Hz, 1H), 3.59 (dd, *J* = 14.1, 4.2 Hz, 1H), 3.53 - 3.45 (m, 1H).

### Synthesis of Intermediate VI

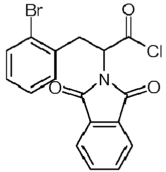

Intermediate VI was prepared following Method B from Intermediate V (1.1 g, 1 eq) in toluene (30 mL) at reflux, to yield expected product (quantitative yield assumed).

### Synthesis of Intermediate VII

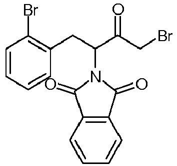

Intermediate VII was prepared following Method C from Intermediate VI (1.15 g, 1 eq) in THF (20 mL); AcOH (15 mL) to yield 1.14 g, 86%, red oily-solid (TLC: 30% EtOAc in cHex).

### Synthesis of Intermediate VIII

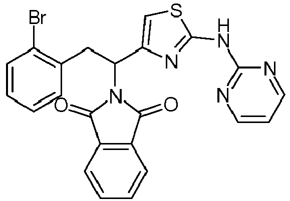

Intermediate VIII was prepared following Method D from Intermediate VII (1.14 g, 1 eq) and 1-(pyrimidin-2-yl)thiourea (390 mg, 1 eq) in DMF (25 mL) to yield 1.07 g, 83%, beige solid.
HPLC-MS (method 4): Rt = 4.8 min [M+H]+ 506.0/508.0.
1H NMR (300 MHz, DMSO) δ 11.64 (s, 1H), 8.62 (d, *J* = 4.9 Hz, 2H), 7.79 (m, 4H), 7.56 (d, *J* = 7.3 Hz, 1H), 7.21 (s, 1H), 7.18 - 7.05 (m, 3H), 7.00 (t, *J* = 4.8 Hz, 1H), 5.75 - 5.62 (m, 1H), 3.82 - 3.68 (m, 2H).

### Synthesis of Intermediate IX

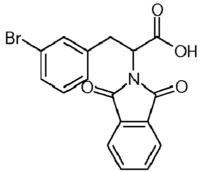

Intermediate IX was prepared following Method A from 3-bromo-DL-phenylalanine (750 mg, 1 eq) and phthalic anhydride (455 mg, 1 eq) in acetonitrile (8 mL) to yield 1.17 g, 100%, white solid.
¹H NMR (300 MHz, DMSO) δ 7.91 - 7.80 (m, 4H), 7.41 (s, 1H), 7.36 - 7.25 (m, 1H), 7.19 - 7.08 (m, 2H), 5.15 (dd, *J* = 11.5, 4.9 Hz, 1H), 3.48 (dd, *J* = 14.1, 4.9 Hz, 1H), 3.32 - 3.21 (m, 1H).

### Synthesis of Intermediate X

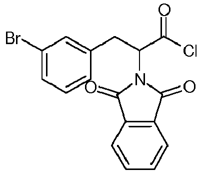

Intermediate X was prepared following Method B from Intermediate IX (1.18 g, 1 eq) in toluene (32 mL) at reflux, to yield expected product (quantitative yield assumed).

### Synthesis of Intermediate XI

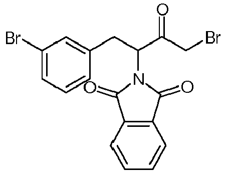

Intermediate XI was prepared following Method C from Intermediate X (1.24 g, 1 eq) in THF (21 mL); AcOH (16 mL) to yield 1.27 g, 89%, orange oily-solid (TLC: 30% EtOAc in cHex).

### Synthesis of Intermediate XII

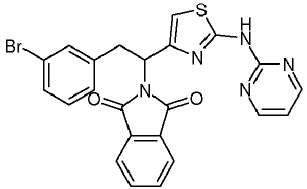

Intermediate XII was prepared following Method D from Intermediate XI (1.27 g, 1 eq) and 1-(pyrimidin-2-yl)thiourea (434 mg, 1 eq) in DMF (28 mL) to yield 1.23 g, 86%, off-white solid.
HPLC-MS (method 4): Rt = 4.7 min [M+H]+ 506.0/508.0.
1H NMR (300 MHz, DMSO) δ 11.65 (s, 1H), 8.62 (d, *J* = 4.9 Hz, 2H), 7.85 - 7.76 (m, 4H), 7.45 (s, 1H), 7.35 - 7.26 (m, 1H), 7.25 - 7.08 (m, 3H), 7.01 (t, *J* = 4.8 Hz, 1H), 5.67 - 5.47 (m, 1H), 3.76 - 3.52 (m, 2H).

### Synthesis of Intermediate XIII

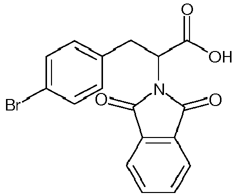

Intermediate XIII was prepared following Method A from 4-bromo-DL-phenylalanine (750 mg, 1 eq) and phthalic anhydride (455 mg, 1 eq) in acetonitrile (8 mL) to yield 1.17 g, 100%, white solid.
¹H NMR (300 MHz, DMSO) δ 7.85 (s, 4H), 7.37 (d, *J* = 8.4 Hz, 2H), 7.12 (d, *J* = 8.4 Hz, 2H), 5.12 (dd, *J* = 11.5, 4.9 Hz, 1H), 3.45 (dd, *J* = 14.1, 4.9 Hz, 1H), 3.32 - 3.24 (m, 1H).

### Synthesis of Intermediate XIV

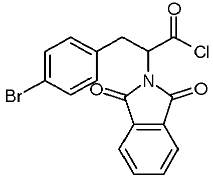

Intermediate XIV was prepared following Method B from Intermediate XIII (1.17 g, 1 eq) in toluene (32 mL) at reflux, to yield expected product (quantitative yield assumed).

### Synthesis of Intermediate XV

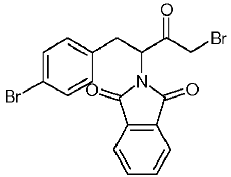

Intermediate XV was prepared following Method C from Intermediate XIV (1.23 g, 1 eq) in THF (21 mL); AcOH (16 mL) to yield 1.3 g, 92%, beige solid (TLC: 30% EtOAc in cHex).

### Synthesis of Intermediate XVI

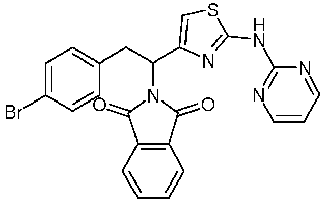

Intermediate XVI was prepared following Method D from Intermediate XV (1.3 g, 1 eq) and 1-(pyrimidin-2-yl)thiourea (444 mg, 1 eq) in DMF (30 mL) to yield 1.38 g, 94%, yellow solid.
HPLC-MS (method 4): Rt = 4.8 min [M+H]+ 506.0/508.0.
1H NMR (300 MHz, DMSO) δ 11.63 (s, 1H), 8.59 (d, *J* = 4.8 Hz, 2H), 7.81 (s, 4H), 7.39 (d, *J* = 8.4 Hz, 2H), 7.17 (d, *J* = 8.4 Hz, 2H), 7.14 (s, 1H), 7.00 (t, *J* = 4.8 Hz, 1H), 7.01 (m, 1H), 5.60 - 5.50 (m, 1H), 3.65 - 3.57 (m, 2H).

### Synthesis of Intermediate XXI

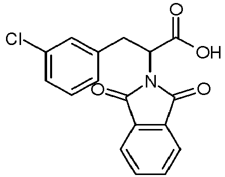

Intermediate XXI was prepared following Method A from 3-chloro-DL-phenylalanine (200 mg, 1 eq) and phthalic anhydride (148 mg, 1 eq) in acetonitrile (2 mL) to yield 329 mg, 100%, white solid.
1H NMR (300 MHz, DMSO) δ 13.40 (s, 1H), 7.85 (s, 4H), 7.28 (s, 1H), 7.21 - 7.13 (m, 2H), 7.11 - 7.07 (m, 1H), 5.17 (dd, *J* = 11.5, 4.9 Hz, 1H), 3.48 (dd, *J* = 14.1, 4.8 Hz, 1H), 3.43 - 3.38 (m, 1H).

### Synthesis of Intermediate XXII

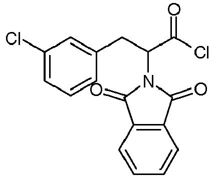

Intermediate XXII was prepared following Method B from Intermediate XXI (329 mg, 1 eq) in toluene (10 mL) at reflux, to yield expected product (quantitative yield assumed).

### Synthesis of Intermediate XXIII

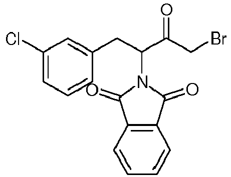

Intermediate XXIII was prepared following Method C from Intermediate XXII (348 mg, 1 eq) in THF (10 mL); AcOH (4 mL) to yield 406 mg, 100%, brown solid (TLC: 30% EtOAc in cHex).

### Synthesis of Intermediate XXIV

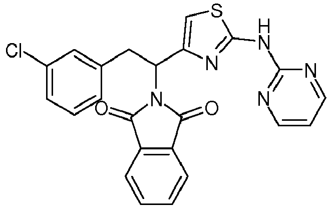

Intermediate XXIV was prepared following Method D from Intermediate XXIII (406 mg, 1 eq) and 1-(pyrimidin-2-yl)thiourea (154 mg, 1 eq) in DMF (10 mL) to yield 226 mg, 49%, white solid.
HPLC-MS (method 4): Rt = 4.7 min [M+H]+ 462.2.

### Synthesis of Intermediate XXXIII

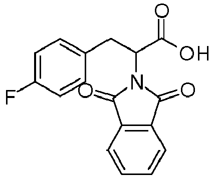

Intermediate XXXIII was prepared following Method A from 4-fluoro-DL-phenylalanine (2 g, 1 eq) and phthalic anhydride (1.6 g, 1 eq) in acetonitrile (22 mL) to yield 3.42 g, 100%, off-white solid.
HPLC-MS (method 4): Rt = 3.9 min; no ionization.

### Synthesis of Intermediate XXXIV

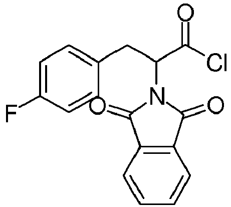

Intermediate XXXIV was prepared following Method B from Intermediate XXXIII (3.42 g, 1 eq) in toluene (110 mL) at reflux to yield expected product (quantitative yield assumed).

### Synthesis of Intermediate XXXV

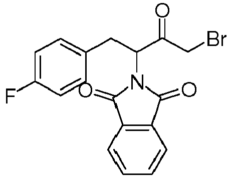

Intermediate XXXV was prepared following Method C from Intermediate XXXIV (3.62 g, 1 eq) in THF (110 mL); AcOH (54 mL) to yield 3.72 g, 87%, brown oily-solid (TLC: 30% EtOAc in cHex).

### Synthesis of Intermediate XXXVI

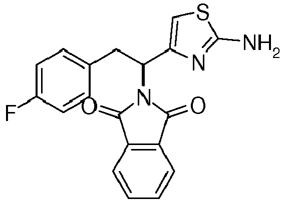

Intermediate XXXVI was prepared following Method D from Intermediate XXXV (3.76 g, 1 eq) and thiourea (734 mg, 1 eq) in DMF (96 mL) to yield 1.58 g, 45%, beige solid. HPLC-MS (method 4): Rt = 3.6 min [M+H]+ 368.2.
¹H NMR (300 MHz, DMSO) δ 7.79 (s, 4H), 7.20 (dd, *J* = 8.5, 5.6 Hz, 2H), 7.01 (m, 4H), 6.60 (s, 1H), 5.35 (t, *J* = 8.2 Hz, 1H), 3.53 (m, 2H).

### Synthesis of Intermediate XXXVII

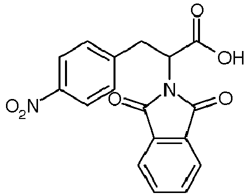

Intermediate XXXVII was prepared following Method A from 4-nitro-DL-phenylalanine (1 g, 1 eq) and phthalic anhydride (705 mg, 1 eq) in acetonitrile (12 mL) to yield 1.63 g, 100%, beige solid.
¹H NMR (300 MHz, DMSO) δ 8.06 (d, *J* = 8.8 Hz, 2H), 7.84 (s, 4H), 7.48 (d, *J* = 8.8 Hz, 2H), 5.23 (dd, *J* = 11.3, 4.9 Hz, 1H), 3.62 (dd, *J* = 14.1, 4.9 Hz, 1H), 3.46 (dd, *J* = 14.1, 11.4 Hz, 1H).

### Synthesis of Intermediate XXXVIII

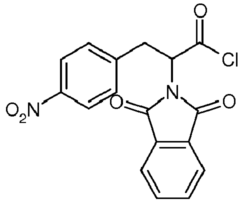

Intermediate XXXVIII was prepared following Method B from Intermediate XXXVII (1.63 g, 1 eq) in toluene (50 mL) at reflux, to yield expected product (quantitative yield assumed).

### Synthesis of Intermediate XXXIX

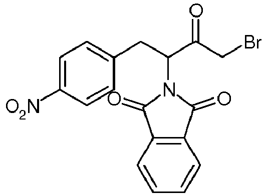

Intermediate XXXIX was prepared following Method C from Intermediate XXXVIII (1.7 g, 1 eq) in THF (32 mL); AcOH (24 mL) to yield 1.95 g, 98%, beige solid (TLC: 30% EtOAc in cHex).

### Synthesis of Intermediate XL

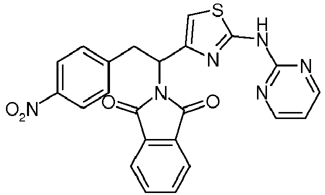

Intermediate XL was prepared following Method D from Intermediate XXXIX (1.95 g, 1 eq) and 1-(pyrimidin-2-yl)thiourea (720 mg, 1 eq) in DMF (45 mL) to yield 2.1 g, 95%, beige solid.
1H NMR (300 MHz, DMSO) δ 11.50 (s, 1 H), 8.45 (d, *J* = 4.8 Hz, 2H), 7.93 (d, *J* = 8.8 Hz, 2H), 7.65 (s, 4H), 7.38 (d, *J* = 8.8 Hz, 2H), 7.03 (s, 1H), 6.85 (t, *J* = 4.8 Hz, 1H), 5.50 (dd, *J* = 10.6, 5.5 Hz, 1H), 3.77 - 3.53 (m, 2H).

### Synthesis of Intermediate XLIX

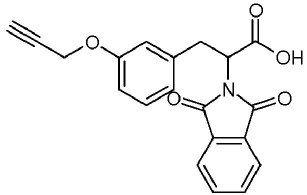

Intermediate XLIX was prepared following Method A from Intermediate XCIX (370 mg, 1 eq) and phthalic anhydride (250 mg, 1 eq) in acetonitrile (4.3 mL) to yield 590 mg, 100%, orange solid, as a mixture of the O-alkylated and the -OH compounds.

### Synthesis of Intermediate L

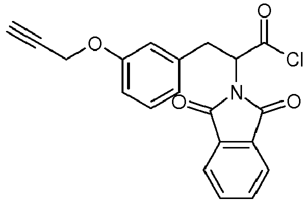

Intermediate L was prepared following Method B from Intermediate XLIX (590 mg, 1 eq) in toluene (17 mL) at reflux to yield the expected products (quantitative yield assumed).

### Synthesis of Intermediate LI

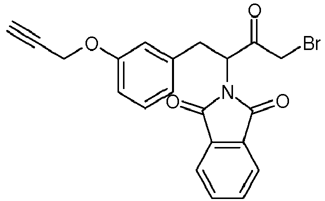

Intermediate LI was prepared following Method C from Intermediate L (620 mg, 1 eq) in THF (12 mL); AcOH (9 mL) to yield 640 mg, 89%, red oil, as a mixture of the O-alkylated and the -OH compounds (TLC: 30% EtOAc in cHex showed 2 spots).

### Synthesis of Intermediate LII

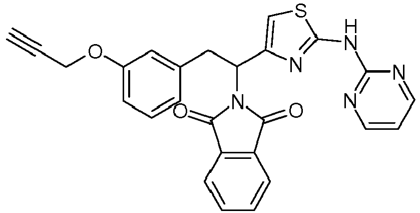

Intermediate LII was prepared following Method D from Intermediate LI (640 mg, 1 eq) and 1-(pyrimidin-2-yl)thiourea (232 mg, 1 eq) in DMF (15 mL) to yield the expected product together with the OH-derivative, 490 mg, 67%, orange solid.
HPLC-MS (method 4): Rt = 4.2 min [M+H]+ 444.1 and 4.6 min [M+H]+ 482.1.

### Synthesis of Intermediate LIII

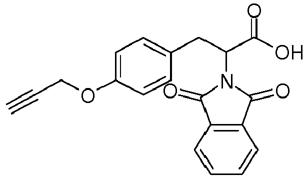

Intermediate LIII was prepared following Method A from Intermediate CIV (540 mg, 1 eq) and phthalic anhydride (365 mg, 1 eq) in acetonitrile (6.2 mL) to yield 860 mg, 100%, yellow foam.
¹H NMR (300 MHz, DMSO) δ 7.88 - 7.82 (m, 4H), 7.07 (d, *J* = 8.6 Hz, 2H), 6.78 (d, *J* = 8.6 Hz, 2H), 5.07 (dd, *J* = 11.6, 4.9 Hz, 1H), 4.67 (d, *J* = 2.4 Hz, 2H), 3.49 (t, *J* = 2.3 Hz, 1H), 3.44 - 3.38 (m, 1H), 3.32 - 3.23 (m, 1H).

### Synthesis of Intermediate LIV

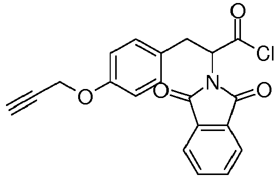

Intermediate LIV was prepared following Method B from Intermediate LIII (860 mg, 1 eq) in toluene (25 mL) at reflux to yield the expected product (quantitative yield assumed).

### Synthesis of Intermediate LV

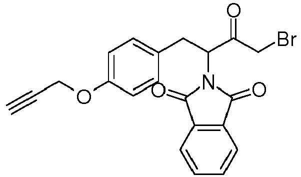

Intermediate LV was prepared following Method C from Intermediate LIV (900 mg, 1 eq) in THF (16 mL); AcOH (12 mL) to yield 940 mg, 90%, brown oily-solid (TLC: 30% EtOAc in cHex).

### Synthesis of Intermediate LVI

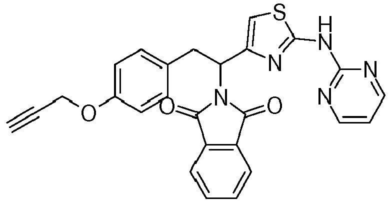

Intermediate LVI was prepared following Method D from Intermediate LV (940 mg, 1 eq) and 1-(pyrimidin-2-yl)thiourea (340 mg, 1 eq) in DMF (22 mL) to yield 2.1 g, 100%, beige solid.
HPLC-MS (method 4): Rt = 4.7 min [M+H]+ 482.1.

### Synthesis of Intermediate LXI

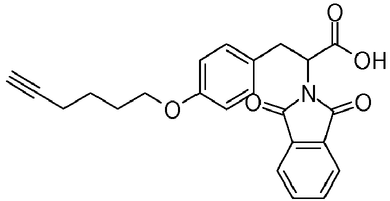

Intermediate LXI was prepared following Method A from Intermediate CX (170 mg, 1 eq) and phthalic anhydride (96 mg, 1 eq) in acetonitrile (2 mL) to yield 255 mg, 100%, beige solid.
¹H NMR (300 MHz, DMSO) δ 7.84 (s, 4H), 7.03 (d, *J* = 8.6 Hz, 2H), 6.71 (d, *J* = 8.6 Hz, 2H), 5.05 (dd, *J* = 11.6, 4.9 Hz, 1H), 3.83 (t, *J* = 6.3 Hz, 2H), 3.28 - 3.20 (m, 2H), 2.74 (t, *J* = 2.2 Hz, 1H), 2.17 (td, *J* = 6.9, 2.5 Hz, 2H), 1.76 - 1.63 (m, 2H), 1.63 - 1.45 (m, 2H).

### Synthesis of Intermediate LXII

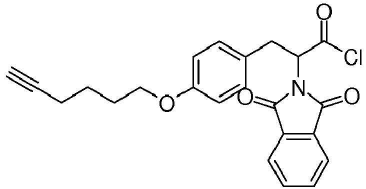

Intermediate LXII was prepared following Method B from Intermediate LXI (255 mg, 1 eq) in toluene (7 mL) at reflux to yield the expected product (quantitative yield assumed).

### Synthesis of Intermediate LXIII

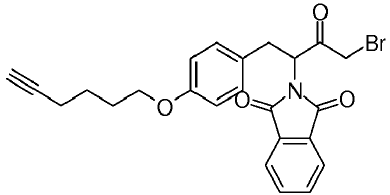

Intermediate LXIII was prepared following Method C from Intermediate LXII (265 mg, 1 eq) in THF (5 mL); AcOH (3.5 mL) to yield 300 mg, 100%, orange oily-solid (TLC: 30% EtOAc in cHex).

### Synthesis of Intermediate LXIV

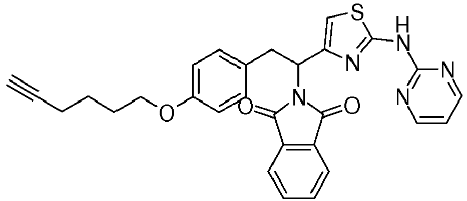

Intermediate LXIV was prepared following Method D from Intermediate LXIII (300 mg, 1 eq) and 1-(pyrimidin-2-yl)thiourea (100 mg, 1 eq) in DMF (6.5 mL) to yield 400 mg, 100%, yellow solid.

### Synthesis of Intermediate LXV

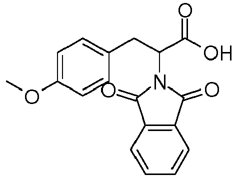

Intermediate LXV was prepared following Method A from 4-methoxy-DL-phenylalanine (500 mg, 1 eq) and phthalic anhydride (379 mg, 1 eq) in acetonitrile (10 mL) to yield 840 mg, 100%, yellow solid.
¹H NMR (300 MHz, DMSO) δ 7.84 (s, 4H), 7.05 (d, *J* = 8.6 Hz, 2H), 6.72 (d, *J* = 8.6 Hz, 2H), 5.06 (dd, *J* = 11.6, 4.9 Hz, 1H), 3.62 (s, 3H), 3.33 (m, 2H).

### Synthesis of Intermediate LXVI

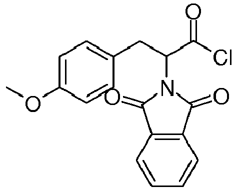

Intermediate LXVI was prepared following Method B from Intermediate LXV (840 mg, 1 eq) in toluene (25 mL) at reflux to yield the expected product (quantitative yield assumed).

### Synthesis of Intermediate LXVII

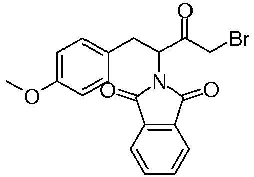

Intermediate LXVII was prepared following Method C from Intermediate LXVI (900 mg, 1 eq) in THF (18 mL); AcOH (13 mL) to yield 970 mg, 100%, orange oil (TLC: 30% EtOAc in cHex).

### Synthesis of Intermediate LXVIII

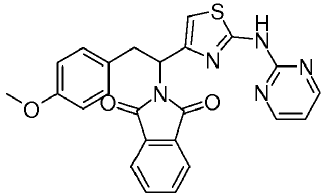

Intermediate LXVIII was prepared following Method D from Intermediate LXVII (970 mg, 1 eq) and 1-(pyrimidin-2-yl)thiourea (372 mg, 1 eq) in DMF (24 mL) to yield 1.03 g, 93%, clear yellow solid.
HPLC-MS (method 4): Rt = 4.4 min [M+H]+ 458.1.
1H NMR (300 MHz, DMSO) δ 11.65 (s, 1H), 8.60 (d, *J* = 4.8 Hz, 2H), 7.80 (s, 4H), 7.14 (s, 1H), 7.11 (d, *J* = 8.6 Hz, 2H), 7.00 (t, *J* = 4.8 Hz, 1H), 6.75 (d, *J* = 8.6 Hz, 2H), 5.52 (m, 1H), 3.63 (s, 3H), 3.57 (m, 2H).

### Synthesis of Intermediate LXIX

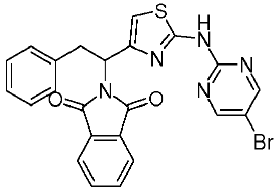

Intermediate LXIX was prepared following Method D from Intermediate III (400 mg, 1 eq) and (5-bromo-pyrimidin-2-yl)-thiourea (250 mg, 1 eq) in DMF (10 mL) to yield 530 mg, 97%, white solid.
HPLC-MS (method 4): Rt = 5.0 min [M+H]+ 506.0/508.0.
¹H NMR (300 MHz, DMSO) δ 11.89 (s, 1H), 8.78 (s, 2H), 7.83 - 7.76 (m, 4H), 7.19 - 7.10 (m, 6H), 5.61 - 5.51 (m, 1H), 3.65 - 3.54 (m, 2H).

### Synthesis of Intermediate LXX

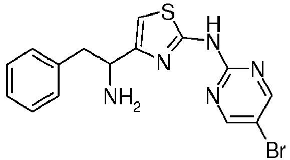

Intermediate LXX was synthesized following Method E by reaction of Intermediate LXIX (480 mg, 1 eq) and methylhydrazine (1 mL, 20 eq) in EtOH (20 mL) at 80 ºC for 17 h to yield 190 mg, 53%, white solid.
HPLC-MS (method 4): Rt = 2.1 min [M+H]+ 376.0/378.0.
1H NMR (300 MHz, DMSO) δ 8.77 (s, 2H), 7.34 - 7.07 (m, 5H), 6.80 (d, *J* = 0.7 Hz, 1H), 4.08 (dd, *J* = 7.4, 5.7 Hz, 1H), 3.12 (dd, *J* = 13.2, 5.5 Hz, 1H), 2.80 (dd, *J* = 13.2, 7.8 Hz, 1H).

### Synthesis of Intermediate LXXXVII

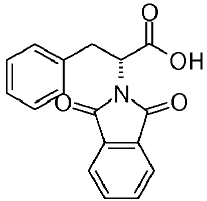

Intermediate LXXXVII was prepared following Method A from D-phenylalanine (1 g, 1 eq) and phthalic anhydride (897 mg, 1 eq) in acetonitrile (12 mL) to yield 1.8 g, 100%, white solid.
¹H NMR (300 MHz, DMSO) δ 7.83 (s, 4H), 7.22 - 7.05 (m, 5H), 5.11 (dd, *J* = 11.6, 4.9 Hz, 1H), 3.48 (dd, *J* = 14.0, 4.9 Hz, 1H), 3.36 (m, 1H).

### Synthesis of Intermediate LXXXVIII

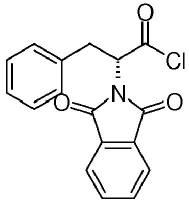

Intermediate LXXXVIII was prepared following Method B from Intermediate LXXXVII (400 mg, 1 eq) in toluene (14 mL) at reflux to yield the expected product (quantitative yield assumed).

### Synthesis of Intermediate LXXXIX

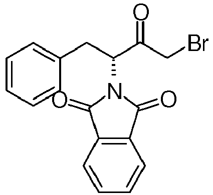

Intermediate LXXXIX was prepared following Method C from Intermediate LXXXVIII (423 mg, 1 eq) in THF (14 mL); AcOH (7 mL) to yield 356 mg, 71%, orange oily-solid (TLC: 30% EtOAc in cHex).

### Synthesis of Intermediate XC

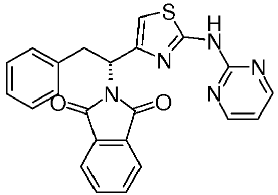

Intermediate XC was prepared following Method D from Intermediate LXXXIX (196 mg, 1 eq) and 1-(pyrimidin-2-yl)thiourea (81 mg, 1 eq) in DMF (5 mL) to yield 170 mg, 75%, beige solid.
HPLC-MS (method 4): Rt = 5.7 min [M+H]+ 428.2.
1H NMR (300 MHz, DMSO) δ 11.64 (s, 1H), 8.61 (d, *J* = 4.8 Hz, 2H), 7.80 (s, 4H), 7.22 - 7.15 (m, 6H), 7.01 (t, *J* = 4.8 Hz, 1H), 5.63 - 5.53 (m, 1H), 3.68 - 3.61 (m, 2H).

### Synthesis of Intermediate XCV

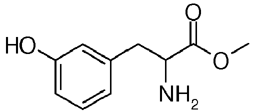

HCI salt of Intermediate XCV was prepared following Method G from DL-m-Tyrosine (300 mg, 1 eq) to yield 370 mg, 96%, off-white solid.
1H NMR (300 MHz, DMSO) δ 9.54 (bs, 1H), 8.65 (s, 3H), 7.10 (t, *J* = 8.0 Hz, 1H), 6.73 - 6.65 (m, 1H), 6.62 (m, 2H), 4.18 (m, 1H), 3.67 (s, 3H), 3.09 (dd, *J* = 13.9, 5.7 Hz, 1H), 3.04 - 2.89 (m, 1H).

### Synthesis of Intermediate XCVI

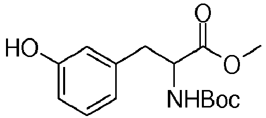

Intermediate XCVI was prepared following Method H from Intermediate XCV (370 mg, 1 eq) in dioxane:H₂O (1:1, 6 mL) by reaction with NaHCO₃ (400 mg, 3 eq) and Boc₂O (0.5 mL, 1.2 eq) to yield 500 mg, quantitative, off-white solid.
1H NMR (300 MHz, DMSO) δ 9.31 (s, 1H), 7.27 (t, *J* = 6.7 Hz, 1H), 7.07 (m, 1H), 6.71 - 6.53 (m, 3H), 4.20 - 4.06 (m, 1H), 3.60 (s, 3H), 2.88 (dd, *J* = 13.7, 5.1 Hz, 1H), 2.83 - 2.67 (m, 1H), 1.33 (s, 9H).

### Synthesis of Intermediate XCVII

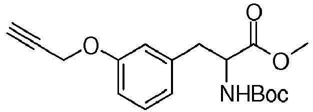

Intermediate XCVII was prepared following Method I from Intermediate XCVI (500 mg, 1 eq), propargyl bromide (0.3 mL, 1.5 eq) and K₂CO₃ (600 mg, 2.5 eq) in DMF (9 mL) at rt for 2 h to give a mixture of Intermediate XCVII and the starting material, Intermediate XCVI; 600 mg, quant., beige solid.
HPLC-MS (method 4): Rt = 4.4 min [M+H-BOC]+ 234.2 and Rt = 4.7 min [M+H-BOC]+ 196.2 (starting material).

### Synthesis of Intermediate XCVIII

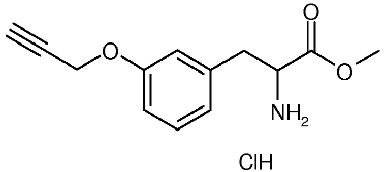

Intermediate XCVIII was prepared following Method J from Intermediate XCVII (560 mg, 1 eq) in 4N HCI in dioxane (12 mL) to yield the resulting hydrochloric salt.

### Synthesis of Intermediate XCIX

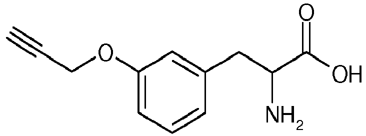

Intermediate XCIX was prepared following Method K from Intermediate XCVIII (450 mg, 1 eq) in MeOH (2 mL) and NaOH (200 mg, 3 eq) in H₂O (3.3 mL) stirring at rt for 1.5 h to yield the expected product (quant. yield assumed, beige solid).

### Synthesis of Intermediate C

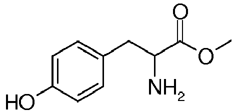

HCI salt of Intermediate C was prepared following Method G from DL-tyrosine (1 g, 1 eq) to yield 1.08 g, 100%, off-white solid.
1H NMR (300 MHz, DMSO) δ 9.45 (s, 1H), 8.54 (bs, 3H), 7.06 - 6.94 (m, 2H), 6.78 - 6.64 (m, 2H), 4.17 (t, J= 6.3 Hz, 1H), 3.67 (s, 3H), 3.05 - 3.00 (m, 2H).

### Synthesis of Intermediate CI

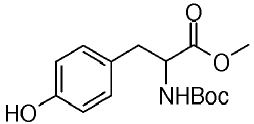

Intermediate CI was prepared following Method H from Intermediate C (1.08 g, 1 eq) in dioxane:H₂O (1:1, 20 mL) by reaction with NaHCO₃ (1.4 g, 3 eq) and Boc₂O (1.52 mL, 1.2 eq) to yield 1.7 g, quantitative, off-white solid.
1H NMR (300 MHz, DMSO) δ 9.22 (s, 1H), 7.24 (d, *J* = 8.0 Hz, 1H), 7.00 (d, *J* = 8.4 Hz, 2H), 6.65 (d, *J* = 8.4 Hz, 2H), 4.05 (m, 1H), 3.58 (s, 3H), 2.84 (dd, *J* = 13.7, 5.3 Hz, 1H), 2.72 (dd, *J* = 13.8, 9.8 Hz, 1H), 1.29 (s, 9H).

### Synthesis of Intermediate CII

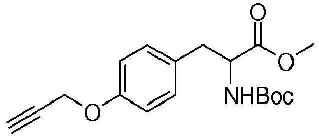

Intermediate CII was prepared following Method I from Intermediate CI (800 mg, 1 eq), propargyl bromide (0.44 mL, 1.5 eq) and K₂CO₃ (940 mg, 2.5 eq) in DMF (14 mL) at rt for 20 h to yield 980 mg, quantitative, yellow solid.
1H NMR (300 MHz, CDCl3) δ 7.05 (d, *J* = 8.5 Hz, 2H), 6.90 (d, *J* = 8.7 Hz, 2H), 4.67 (d, *J* = 2.4 Hz, 2H), 4.54 (m, 1H), 3.71 (s, 3H), 3.05 (m, 2H), 2.52 (t, *J* = 2.4 Hz, 1H), 1.42 (s, 9H).

### Synthesis of Intermediate CIII

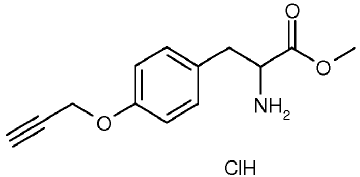

Intermediate CIII was prepared following Method J from Intermediate CII (910 mg, 1 eq) in 4N HCI in dioxane (7 mL) to yield the resulting hydrochloric salt.
1H NMR (300 MHz, DMSO) δ 8.63 (bs, 3H), 7.19 (d, *J* = 10.1 Hz, 2H), 6.97 (d, *J* = 10.1 Hz, 2H), 4.78 (d, *J* = 2.4 Hz, 2H), 4.22 (m, 1H), 3.71 (s, 3H), 3.58 (t, *J* = 2.4 Hz, 1H), 3.08 (m, 2H).

### Synthesis of Intermediate CIV

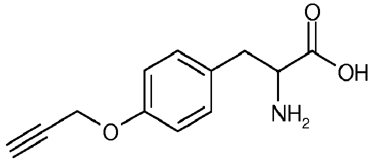

Intermediate CIV was prepared following Method K from Intermediate CIII (740 mg, 1 eq) in MeOH (3 mL) and NaOH (330 mg, 3 eq) in H₂O (5 mL) at rt for 18 h to yield 540 mg, 89%, beige solid.
1H NMR (300 MHz, DMSO) δ 7.19 (d, *J* = 8.6 Hz, 2H), 6.90 (d, *J* = 8.6 Hz, 2H), 4.75 (d, *J* = 2.4 Hz, 2H), 3.55 (t, *J* = 2.3 Hz, 1H), 3.35 (m, 1H), 3.08 (dd, *J* = 14.4, 4.2 Hz, 1H), 2.85 - 2.72 (m, 1H).

### Synthesis of Intermediate CVIII

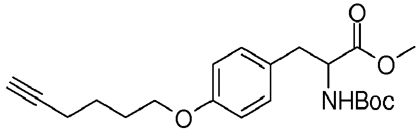

Intermediate CVIII was prepared following Method I from Intermediate CI (200 mg, 1 eq), 6-iodo-1-hexyne (211 mg, 1.5 eq) and K₂CO₃ (234 mg, 2.5 eq) in DMF (4 mL) heating at 50 ºC for 17 h to yield 215 mg, 84%, white solid.
HPLC-MS (method 4): Rt = 4.7 min [M+H-BOC]+ 276.2.
1H NMR (300 MHz, DMSO) δ 7.29 (t, J= 9.5 Hz, 1H), 7.10 (t, *J=* 9.1 Hz, 2H), 6.82 (d, *J* = 8.6 Hz, 2H), 4.08 (td, *J* = 9.8, 5.3 Hz, 1H), 3.93 (t, *J* = 6.3 Hz, 2H), 3.60 (s, 3H), 2.89 (dd, *J* = 13.8, 5.2 Hz, 1H), 2.82 - 2.68 (m, 2H), 2.26 - 2.15 (m, 2H), 1.86 - 1.68 (m, 2H), 1.66 - 1.51 (m, 2H), 1.29 (s, 9H).

### Synthesis of Intermediate CIX

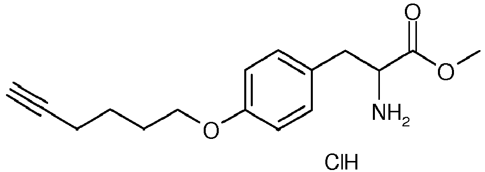

Intermediate CIX was prepared following Method J from Intermediate CVIII (270 mg, 1 eq) in 4N HCI in dioxane (4 mL) to yield the resulting hydrochloric salt (225 mg, quant., white solid).

### Synthesis of Intermediate CX

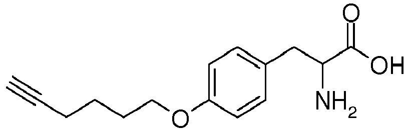

Intermediate CX was prepared following Method K from Intermediate CIX (225 mg, 1 eq) in MeOH (3 mL) and NaOH (87 mg, 3 eq) in H₂O (1.3 mL) stirring at rt for 17 h to yield 170 mg, 90%, white solid.
HPLC-MS (method 4): Rt = 3.0 min [M+H]+ 262.1.
1H NMR (300 MHz, DMSO) δ 7.15 (d, *J* = 8.5 Hz, 2H), 6.83 (d, *J* = 8.6 Hz, 2H), 3.94 (t, *J* = 6.3 Hz, 2H), 3.44 (m, 1H), 3.05 (dd, *J* = 14.2, 4.2 Hz, 1H), 2.85 - 2.68 (m, 2H), 2.23 (m, 2H), 1.86 - 1.69 (m, 2H), 1.67 - 1.49 (m, 2H).

### Synthesis of Intermediate CXI

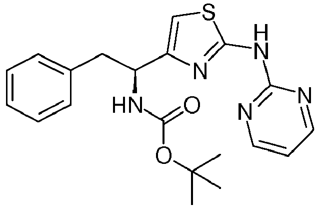

Intermediate CXI was prepared following Method D from (1S)-(1-benzyl-3-bromo-2-oxopropyl)-carbamic acid tert-butyl ester (444 mg, 1 eq) and 1-(pyrimidin-2-yl)thiourea (200 mg, 1 eq) in DMF (13 mL) to yield 500 mg, 97%, white solid.
HPLC-MS (method 4): Rt = 4.6 min [M+H]+ 398.2.

### Synthesis of Intermediate CXII

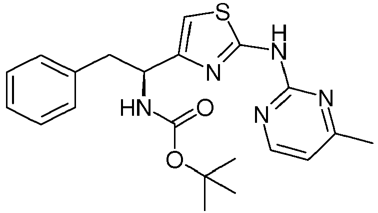

Intermediate CXII was prepared following Method D from (1S)-(1-benzyl-3-bromo-2-oxopropyl)-carbamic acid tert-butyl ester (75 mg, 1 eq) and N-(4-methylpyrimidin-2-yl)thiourea (37 mg, 1 eq) in DMF (2.2 mL) to yield 83 mg, 92%, white solid.
HPLC-MS (method 4): Rt = 4.5 min [M+H]+ 412.2.

### Synthesis of Intermediate CXIII

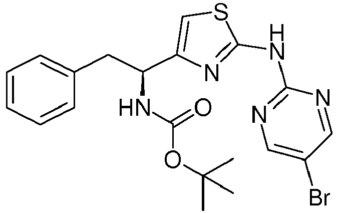

Intermediate CXIII was prepared following Method D from (1S)-(1-benzyl-3-bromo-2-oxo-propyl)-carbamic acid tert-butyl ester (150 mg, 1 eq) and (5-bromo-pyrimidin-2-yl)-thiourea (102 mg, 1 eq) in DMF (4.4 mL) to yield 190 mg, 91%, white solid.
HPLC-MS (method 4): Rt = 4.8 min [M+H]+ 416.0/478.0.
¹H NMR (300 MHz, DMSO) δ 8.77 (s, 2H), 7.31 - 7.14 (m, 5H), 6.80 (s, 1H), 4.77 (m, 1H), 3.18 (m, 1H), 2.81 (m, 1H), 1.31 (s, 9H).

### Synthesis of Intermediate CXIV

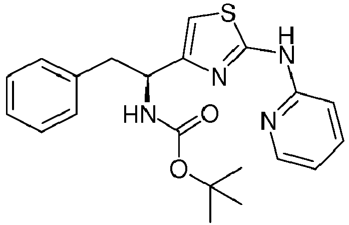

Intermediate CXIV was prepared following Method D from (1S)-(1-benzyl-3-bromo-2-oxo-propyl)-carbamic acid tert-butyl ester (112 mg, 1 eq) and 2-pyridylthiourea (50 mg, 1 eq) in DMF (3.2 mL) to yield 125 mg, 97%, white solid.
HPLC-MS (method 4): Rt = 4.5 min [M+H]+ 397.1.

### Synthesis of Intermediate CXV

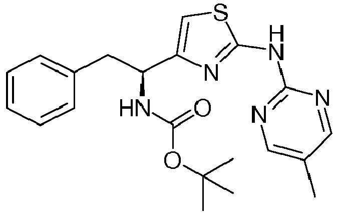

Intermediate CXV was prepared following Method D from (1S)-(1-benzyl-3-bromo-2-oxopropyl)-carbamic acid tert-butyl ester (75 mg, 1 eq) and Intermediate CCV (37 mg, 1 eq) in DMF (2.2 mL) to yield 85 mg, 94%, white solid.
HPLC-MS (method 4): Rt = 4.7 min [M+H]+ 412.1.

### Synthesis of Intermediate CXVI

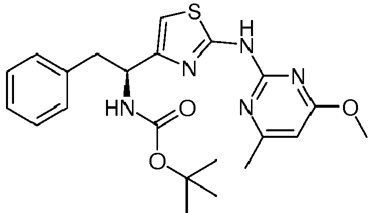

Intermediate CXVI was prepared following Method D from (1S)-(1-benzyl-3-bromo-2-oxo-propyl)-carbamic acid tert-butyl ester (81 mg, 1 eq) and *N*-(4-methoxy-6-methyl-2-pyrimidinyl)thiourea (47 mg, 1 eq) in DMF (2.4 mL) to yield 65 mg, 62%, white solid. HPLC-MS (method 4): Rt = 4.4 min [M+H]+ 442.1.

### Synthesis of Intermediate CXXIII

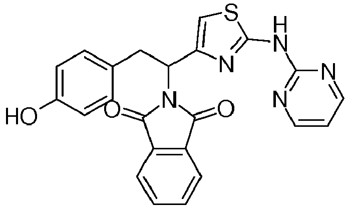

To a suspension of LXVIII (700 mg, 1 eq) in DCM (75 mL) was added BBr3 (1M in DCM (6.12 mL, 4 eq). The mixture was stirred at rt for 3 days. HCl 1M (75 mL) was added and the mixture was extracted with CHCl₃:*ⁱ*PrOH (1:1). The organics were dried, filtered and evaporated to yield 680 mg, quantitative, yellow solid.
HPLC-MS (method 4): Rt = 4.1 min [M+H]+ 444.1.
¹H NMR (300 MHz, DMSO) δ 11.65 (s, 1H), 8.60 (d, *J* = 4.8 Hz, 2H), 7.80 (s, 4H), 7.12 (d, *J* = 1.2 Hz, 1H), 7.01 (t, *J* = 4.8 Hz, 1H), 6.97 (d, *J* = 8.5 Hz, 2H), 6.55 (d, *J* = 8.5 Hz, 2H), 5.47 (dd, *J* = 8.4, 7.0 Hz, 1H), 3.50 (d, *J* = 9.0 Hz, 2H).

### Synthesis of Intermediate CXXXII

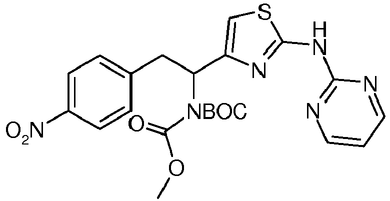

To a suspension of Final Product 14 (180 mg, 1 eq) in THF (6 mL) was added TEA (0.22 mL, 3 eq), DMAP (6 mg, 0.1 eq) and Boc₂O (0.5 mL, 4 eq). The solution was stirred at rt for 3 days. The mixture was concentrated and the residue was purified by chromatography (Biotage, C-20g, 0% to 2% MeOH in DCM) to give 180 mg, 68%, yellow solid).
1H NMR (300 MHz, DMSO) δ 8.76 (d, *J* = 4.8 Hz, 2H), 8.18 (d, *J* = 8.8 Hz, 2H), 7.37 (d, *J* = 8.8 Hz, 2H), 7.12 (t, *J* = 4.8 Hz, 1H), 6.37 (d, *J* = 1.7 Hz, 1H), 5.30 (m, 1H), 3.50 (dd, *J* = 13.6, 3.4 Hz, 1H), 3.41 (dd, *J* = 13.4, 6.8 Hz, 1H), 3.33 (s, 3H), 1.56 (s, 9H).

### Synthesis of Intermediate CXXXIII

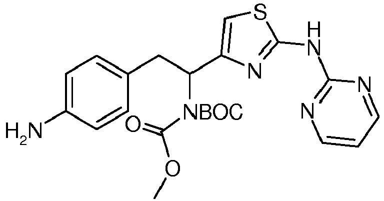

To a solution of Intermediate CXXXII (170 mg, 1 eq) in DCM:MeOH (1:1, 8 mL) was added NH4CI (210 mg, 10 eq) and Zn dust (200 mg, 8 eq). The reaction mixture was stirred at rt for 20 h. The mixture was filtered through celite and rinsed with MeOH. The filtrate was concentrated and the residue was purified by chromatography (Biotage, C-20g, 0% to 2% MeOH in DCM) to yield 90 mg, 56%, white solid.
1H NMR (300 MHz, DMSO) δ 11.71 (s, 1H), 8.61 (d, *J* = 4.8 Hz, 2H), 7.01 (t, *J* = 4.8 Hz, 1H), 6.92 (d, *J* = 1.1 Hz, 1H), 6.87 (d, *J* = 8.3 Hz, 2H), 6.46 (d, *J* = 8.3 Hz, 2H), 5.49 (m, 1H), 4.85 (s, 2H), 3.5 (s, 3H), 3.22 (m, 2H), 1.23 (s, 9H).

### Synthesis of Intermediate CXL

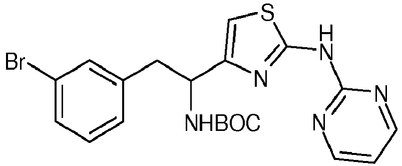

Intermediate CXL was prepared following Method H from Final Compound 5 (650 mg, 1 eq) in dioxane:H₂O (1:1, 20 mL) by reaction with NaHCO₃ (220 mg, 1.5 eq) and Boc₂O (0.5 mL, 1.2 eq) to yield 605 mg, 73%, white solid.
1H NMR (300 MHz, DMSO) δ 11.70 (s, 1H), 8.62 (d, *J* = 4.8 Hz, 2H), 7.43 (s, 1H), 7.38 (m, 1H), 7.25 (m, 3H), 7.02 (m, 1H), 6.79 (s, 1H), 4.76 (m, 1H), 3.19 (m, 1H), 2.84 (m, 1H), 1.30 (s, 9H).

### Synthesis of Intermediate CXLI

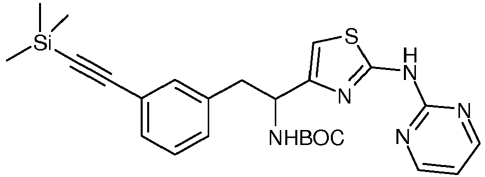

Intermediate CXLI was prepared following Method R by Sonogashira reaction of Intermediate CXL (200 mg, 1 eq) with trimethylsilylacetylene (0.18 mL, 3 eq), PdCl₂(PPh₃)₂ (300 mg, 1 eq), CuI (80 mg, 1 eq), Et₃N (0.4 mL, 7 eq) in DMF (4 mL) at 120 ºC for 5 h. Purification with gradient 10% to 100% EtOAc in cHexane yield 200 mg, 95%, white solid.
HPLC-MS (method 4): Rt = 5.1 min, [M+H]+ 494.1.
1H NMR (300 MHz, DMSO) δ 11.70 (s, 1H), 8.62 (d, *J* = 5.5 Hz, 2H), 7.27 (m, 5H), 7.01 (m, 1H), 6.77 (s, 1H), 4.72 (td, *J* = 9.9, 4.1 Hz, 1H), 3.16 (m, 1H), 2.80 (m, 1H), 1.26 (s, 9H), 0.20 (m, 9H).

### Synthesis of Intermediate CXLII

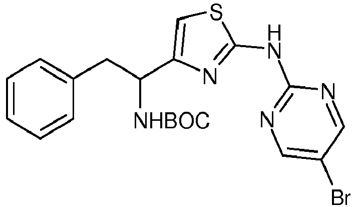

Intermediate CXLII was prepared following Method H from Intermediate LXX (190 mg, 1 eq) in dioxane:H₂O (1:1, 6 mL) by reaction with NaHCO₃ (70 mg, 1.5 eq) and Boc₂O (0.15 mL, 1.2 eq) to yield 230 mg, 95%, white solid.
HPLC-MS (method 4): Rt = 4.9 min, [M+H]+ 476.1/478.1.
1H NMR (300 MHz, DMSO) δ 11.95 (s, 1H), 8.77 (s, 2H), 7.36 - 7.08 (m, 6H), 6.79 (s, 1H), 4.76 (td, *J* = 9.5, 4.5 Hz, 1H), 3.17 (dd, *J* = 13.7, 4.7 Hz, 1H), 2.83 (dd, *J* = 13.6, 10.0 Hz, 1H), 1.23 (m, 9H).

### Synthesis of Intermediate CXLIII

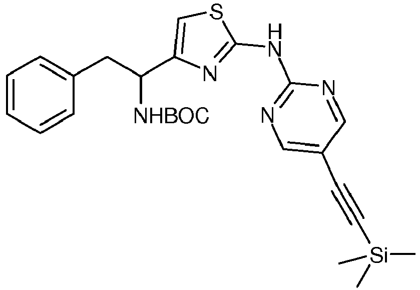

Intermediate CXLIII was prepared following Method R by Sonogashira reaction of Intermediate CXLII (200 mg, 1 eq) with trimethylsilylacetylene (0.18 mL, 3 eq), PdCl₂(PPh₃)₂ (150 mg, 0.5 eq), CuI (40 mg, 0.5 eq), Et₃N (0.41 mL, 7 eq) in DMF (1 mL) at 100 ºC for 24 h. Purification with gradient 10% to 30% EtOAc in cHexane yield 110 mg, 53%, orange solid.
HPLC-MS (method 4): Rt = 5.1 min, [M+H]+ 494.2.
1H NMR (300 MHz, CDCl₃) δ 8.71 (s, 2H), 7.21 - 7.01 (m, 5H), 6.76 (m, 1H), 6.58 (m, 1H), 5.06 (m, 1H), 3.20 (d, *J* = 7.3 Hz, 2H), 1.43 (s, 9H), 0.28 (s, 9H).

### Synthesis of Intermediate CXLV

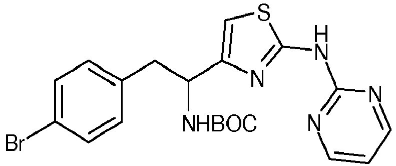

Intermediate CXLV was prepared following Method H from Final Compound 8 (440 mg, 1 eq) in dioxane:H₂O (1:1, 12 mL) by reaction with NaHCO₃ (150 mg, 1.5 eq) and Boc₂O (0.4 mL, 1.2 eq) to yield 600 mg, quantitative yield, white solid.
HPLC-MS (method 4): Rt = 4.9 min, [M+H]+ 476.0/478.0.
1H NMR (300 MHz, DMSO) δ 11.64 (s, 1H), 8.61 (d, *J* = 4.8 Hz, 2H), 7.43 (m, 2H), 7.19 (m, 3H), 7.01 (m, 1H), 6.72 (d, *J* = 15.7 Hz, 1H), 4.73 (m, 1H), 3.16 (m, 1H), 2.82 (m, 1H), 1.30 (s, 9H).

### Synthesis of Intermediate CXLVI

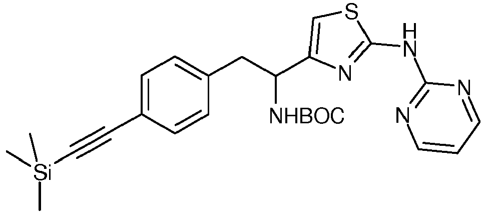

Intermediate CXLVI was prepared following Method R by Sonogashira reaction of Intermediate CXLV (100 mg, 1 eq) with trimethylsilylacetylene (0.09 mL, 3 eq), PdCl₂(PPh₃)₂ (150 mg, 1 eq), CuI (40 mg, 1 eq), Et₃N (0.2 mL, 7 eq) in DMF (2.1 mL) at 120 ºC for 2 h. Purification with gradient 0% to 1% MeOH in DCM yield 75 mg, 50%, orange solid.
HPLC-MS (method 4): Rt = 5.2 min, [M+H]+ 494.2.
¹H NMR (300 MHz, DMSO) δ 11.48 (s, 1H), 8.41 (d, *J* = 4.8 Hz, 2H), 7.30 - 6.90 (m, 5H), 6.80 (t, *J* = 4.8 Hz, 1H), 6.54 (s, 1H), 4.54 (bs, 1H), 2.66 - 2.60 (m, 2H), 1.05 (s, 9H), 0.00 (s, 9H).

### Synthesis of Intermediate CLV

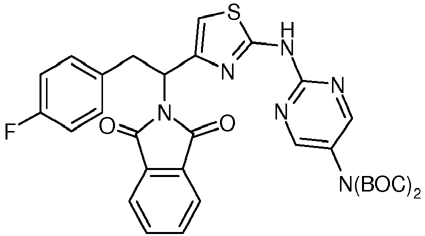

Intermediate XXXVI (100 mg, 1 eq), Imidodicarbonic acid, 2-(2-chloro-5-pyrimidinyl)-, 1,3-bis(1,1-dimethylethyl) ester (CAS: 2137923-49-8; 135 mg, 1.5 eq), Pd₂dba₃ (25 mg, 0.1 eq), Xantphos (31 mg, 0.2 eq) and Cs₂CO₃ (177 mg, 2 eq) were mixed in dioxane (2.7 mL). The mixture was deoxygenated with Argon for few minutes. Then, it was heated at 100 ºC in pressure vial for 3 h. The mixture was taken in ethyl acetate and washed with water. The organic extract was dried and purified by silica flash chromatography (0% to 30% EtOAc in cHex) affording Intermediate CLV, 117 mg, 81%, off-white solid.
HPLC-MS (method 4): Rt = 4.98 min, [M+H]+ 661.1.

### Synthesis of Intermediate CLVI

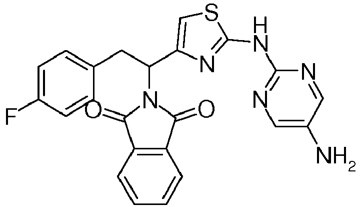

Intermediate CLVI as TFA salt, was prepared following similar conditions that the one used in Method N by treatment of Intermediate CLV (70 mg, 1 eq) in DCM (2 mL) with TFA (0.2 mL, 25 eq) at rt for 2 h. The product was obtained as a trifluoroacetate salt by concentration of the solvents under vacuum (62 mg, 100%) and it was used as such in next synthetic step.

### Synthesis of Intermediate CLVII

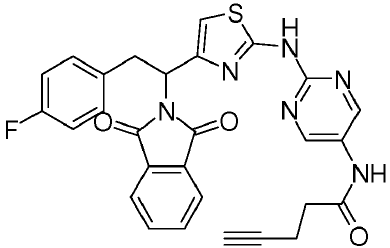

Intermediate CLVII was prepared following Method T by acylation reaction of Intermediate CLVI (62 mg, 1 eq) in DMF (2.2 mL) with DIPEA (0.1 mL, 5 eq), 4-pentynoic acid (21 mg, 2 eq), HOBt (33 mg, 2 eq) and HBTU (82 mg, 2 eq) after stirring for 16 h. Addition of water and filtration afforded Intermediate CLVII, 56 mg, 96%, beige solid.
HPLC-MS (method 4): Rt = 4.4 min, [M+H]+ 541.1.

### Synthesis of Intermediate CLXI

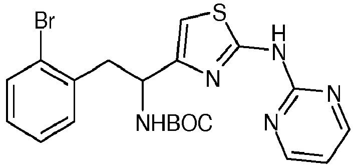

Intermediate CLXI was prepared following Method H from Final Compound 2 (535 mg, 1 eq) in dioxane:H₂O (1:1, 20 mL) by reaction with NaHCO₃ (180 mg, 1.5 eq) and Boc₂O (0.4 mL, 1.2 eq) to give 560 mg, 82%, yellow solid.
HPLC-MS (method 4): Rt = 4.6 min, [M+H]+ 476.1/478.1.
¹H NMR (300 MHz, DMSO) δ 11.70 (s, 1H), 8.61 (dd, J= 8.0, 5.1 Hz, 2H), 7.57 (d, *J* = 7.9 Hz, 1H), 7.33 - 7.20 (m, 3H), 7.15 (dd, *J* = 10.1, 4.5 Hz, 1H), 7.02 (t, *J* = 4.8 Hz, 1H), 6.81 (s, 1H), 4.95 (td, *J* = 9.9, 4.2 Hz, 1H), 3.39 (dd, *J* = 7.8, 3.1 Hz, 1H), 2.91 (dt, *J* = 24.7, 12.2 Hz, 1H), 1.26 (s, 9H).

### Synthesis of Intermediate CLXII

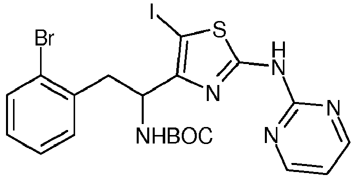

Intermediate CLXII was prepared following Method L from Intermediate CLXI (560 mg, 1 eq) in DMF (12 mL) and NIS (290 mg, 1.1 eq) after 2 h reaction. The crude reaction product was purified by automated chromatography in silica gel (0% to 1% MeOH in DCM) to yield 445 mg, 66%, yellow solid.
HPLC-MS (method 4): Rt = 4.9 min, [M+H]+ 602.0/604.0.

### Synthesis of Intermediate CLXIII

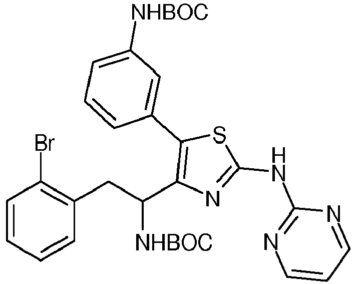

Intermediate CLXXIII was prepared following Method M from Intermediate CLXII (25 mg, 1 eq), (3-boc-aminophenyl)boronic acid (15 mg, 1.5 eq), PdCl₂(PPh₃)₂ (3 mg, 0.1 eq) and Na₂CO₃ 2M (70 □L, 3 eq) in dioxane (0.5 mL). Purification by flash chromatography (0% to 1% MeOH in DCM) gave 20 mg, 72%, beige solid.
HPLC-MS (method 4): Rt = 4.9 min, [M+H]+ 667.2/669.2.

### Synthesis of Intermediate CLXVI

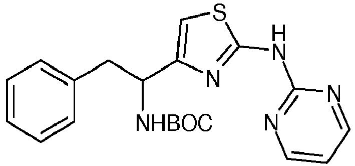

Intermediate CLXVI was prepared following Method H from Final Compound 1 (920 mg, 1 eq) in dioxane:H₂O (1:1, 30 mL) by reaction with NaHCO₃ (390 mg, 1.5 eq) and Boc₂O (0.9 mL, 1.2 eq) to give, after automated chromatography in silica gel (0% to 15% MeOH in DCM), 990 mg, 80%, white solid.
HPLC-MS (method 4): Rt = 4.5 min, [M+H]+ 398.1.
1H NMR (300 MHz, DMSO) δ 11.70 (s, 1H), 8.62 (d, *J* = 4.8 Hz, 2H), 7.34 - 7.09 (m, 6H), 7.01 (t, *J* = 4.8 Hz, 1H), 6.78 (d, *J* = 21.2 Hz, 1H), 4.77 (td, *J* = 9.4, 4.7 Hz, 1H), 3.19 (dd, *J* = 13.7, 4.7 Hz, 1H), 2.84 (dd, *J* = 13.6, 10.0 Hz, 1H), 1.26 (s, 9H).

### Synthesis of Intermediate CLXVII

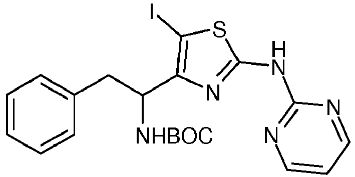

Intermediate CLXVII was prepared following Method L from Intermediate CLXVI (990 mg, 1 eq) in DMF (25 mL) and NIS (620 mg, 1.1 eq) at rt for 2 h to yield 1.24 g, 95%, yellow solid.
HPLC-MS (method 4): Rt = 4.9 min, [M+H]+ 524.1.
1H NMR (300 MHz, DMSO) δ□11.99 (s, 1H), 8.64 (d, *J* = 4.8 Hz, 2H), 7.31 - 6.79 (m, 7H), 4.78 (m, 1H), 2.96 (m, 2H), 1.28 (s, 9H).

### Synthesis of Intermediate CLXVIII

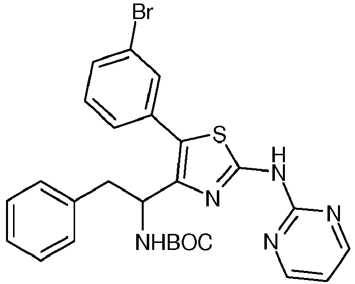

Intermediate CLXVIII was prepared following Method M from Intermediate CLXVII (300 mg, 1 eq), (3-bromophenyl)boronic acid (230 mg, 2 eq), PdCl₂(PPh₃)₂ (80 mg, 0.2 eq) and Na₂CO₃ 2M (0.9 mL, 3 eq) in dioxane (5.7 mL). Purification by flash chromatography (0% to 100% EtOAc in cyclohexane) gave 131 mg, 41%, clear brown solid.
HPLC-MS (method 4): Rt = 5.0 min, [M+H]+ 553.1.

### Synthesis of Intermediate CLXIX

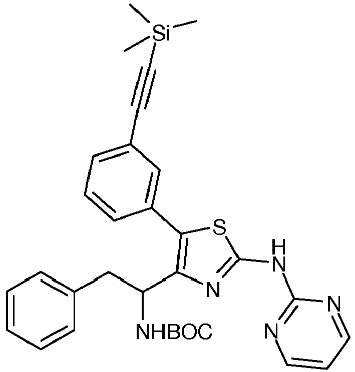

Intermediate CLXIX was prepared following Method R by Sonogashira reaction of CLXVIII (131 mg, 1 eq) with trimethylsilylacetylene (0.1 mL, 3 eq), PdCl₂(PPh₃)₂ (166 mg, 1 eq), CuI (45 mg, 1 eq), Et₃N (0.23 mL, 7 eq) in DMF (2.4 mL) at 120 ºC for 5 h. Purification with gradient 0% to 2% MeOH in DCM gave 83 mg, 61%, clear brown solid.

### Synthesis of Intermediate CLXXII

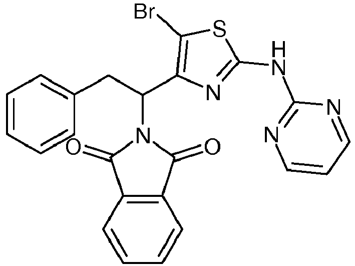

Intermediate CLXXII was prepared following Method L from Intermediate IV (500 mg, 1 eq) in DMF (12 mL) and NBS (250 mg, 1.2 eq) after 3 h reaction to yield 360 mg, 60%, white solid.
HPLC-MS (method 4): Rt = 4.7 min, [M+H]+ 506.0/508.0.

### Synthesis of Intermediate CLXXIII

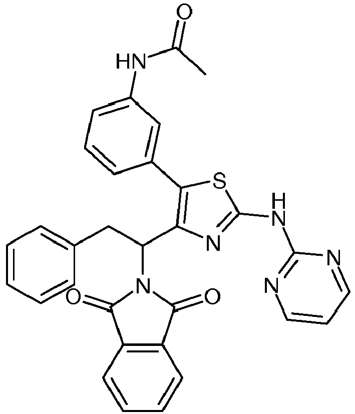

Intermediate CLXXIII was prepared following Method M from Intermediate CLXXII (45 mg, 1 eq), 3-acetamidobenzeneboronic acid (24 mg, 1.5 eq), PdCl₂(PPh₃)₂ (6 mg, 0.1 eq) and Na₂CO₃ 2M (130 □L, 3 eq) in dioxane (1.8 mL). Purification by flash chromatography in silica gel (15% to 100% EtOAc in cHex) gave 20 mg, 40%.
HPLC-MS (method 4): Rt = 4.4 min, [M+H]+ 561.1.

### Synthesis of Intermediate CLXXIV

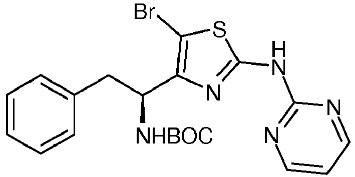

Intermediate CLXXIV was prepared following Method L from Intermediate CXI (250 mg, 1 eq) in acetonitrile (6 mL) and NBS (168 mg, 1.5 eq). After 2 h reaction the crude was purified by automated chromatography in silica gel (0% to 50% EtOAc in cHex) to yield 249 mg, 83%, syrup.
HPLC-MS (method 4): Rt = 4.8 min, [M+H]+ 476.0/478.0.

### Synthesis of Intermediate CLXXV

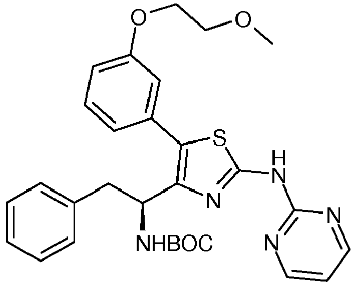

Intermediate CLXXV was prepared following Method M from Intermediate CLXXIV (90 mg, 1 eq), 3-(2-methoxyethoxy)phenylboronic acid (56 mg, 1.5 eq), PdCl₂(PPh₃)₂ (13 mg, 0.1 eq) and Na₂CO₃ 2M (300 □L, 3 eq) in dioxane (2 mL) at 100 ºC for 20 h. Purification by column chromatography in silica (0% to 50% EtOAc in c-Hex) gave 43 mg, 42%.
HPLC-MS (method 4): Rt = 4.8 min, [M+H]+ 548.1.

### Synthesis of Intermediate CLXXVI

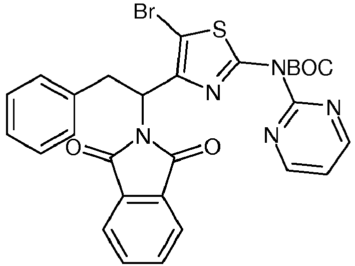

Intermediate CLXXVI was prepared following similar conditions used in Method H by reaction of Intermediate CLXXII (510 mg, 1 eq) in THF (10 mL) by reaction with 4-DMAP(12 mg, 0.1 eq) and Boc₂O (330 mg, 1.5 eq) at reflux for 5h. Purification by automated chromatography in silica gel (0% to 50% EtOAc in cHex) gave 560 mg, 91%, pale pink solid.
1H NMR (300 MHz, CDCl3) δ 8.80 (d, *J* = 4.8 Hz, 2H), 7.71 - 7.58 (m, 4H), 7.30 (t, *J* = 4.8 Hz, 1H), 7.17 - 7.07 (m, 5H), 5.55 (dd, *J* = 10.2, 6.2 Hz, 1H), 3.56 (dd, *J* = 14.0, 10.2 Hz, 1H), 3.41 (dd, *J* = 14.0, 6.2 Hz, 1H), 1.44 (s, 9H).

### Synthesis of Intermediate CLXXVII

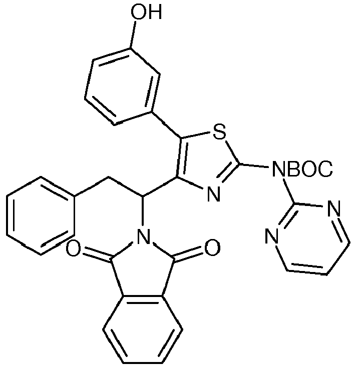

Intermediate CLXXVII was prepared following Method M from Intermediate CLXXVI (550 mg, 1 eq), 3-(4,4,5,5-tetramethyl-1,3,2-dioxaborolan-2-yl)phenol (300 mg, 1.5 eq), PdCl₂(PPh₃)₂ (64 mg, 0.1 eq) and Na₂CO₃ 2M (1.4 mL, 3 eq) in dioxane (9 mL) at 80ºC for 17 h. Purification by chromatography in silica gel (0% to 60% EtOAc in c-Hex) gave 330 mg, 58%, yellow syrup.
HPLC-MS (method 4): Rt = 4.7 min, [M+H]+ 520.1 (fragmentation without BOC).
1H NMR (300 MHz, DMSO) δ 9.62 (s, 1H), 8.77 (d, *J* = 4.9 Hz, 2H), 7.80 - 7.70 (m, 2H), 7.64 - 7.50 (m, 2H), 7.41 (t, *J* = 4.9 Hz, 1H), 7.19 - 7.03 (m, 4H), 7.00 - 6.88 (m, 2H), 6.83 - 6.70 (m, 1H), 6.64 - 6.55 (m, 1H), 5.61 (dd, *J* = 10.7, 5.4 Hz, 1H), 1H missed under water signal, 3.15 (dd, *J* = 13.8, 5.4 Hz, 1H), 1.37 (s, 1H), 1.35 (s, 9H).

### Synthesis of Intermediate CLXXVIII

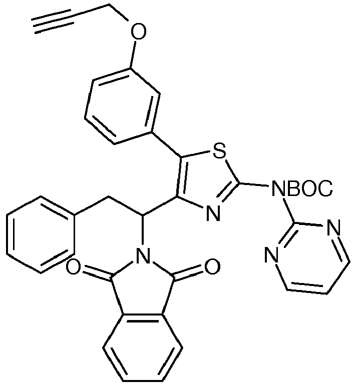

Alkylation of hydroxyl group was afforded following similar conditions used in Method I by reaction of Intermediate CLXXVII (66 mg, 1 eq) in DMF (1 mL) with K₂CO₃ (44 mg, 3 eq) and propargyl bromide (29 □L, 2.5 eq) heating at 80 ºC for 2h. After aqueous work up, Intermediate CLXXVIII was obtained (80 mg, syrup, quant.).
HPLC-MS (method 4): Rt = 4.9 min, [M+H]+ 658.1.

### Synthesis of Intermediate CLXXIX

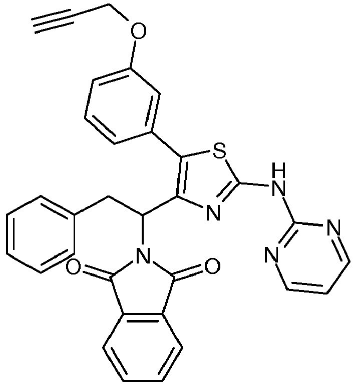

Intermediate CLXXIX was prepared following similar conditions used in Method N by treatment of Intermediate CLXXVIII (70 mg, 1 eq) in DCM (2 mL) with TFA (0.16 mL, 20 eq) at rt for 3 h. The product was obtained as trifluoroacetate salt by concentration of the solvents under vacuum (72 mg, 100%, yellow oil) and it was used as such in the next synthetic step.

### Synthesis of Intermediate CCI

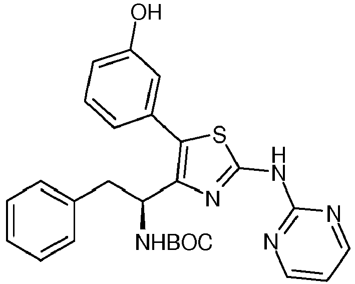

Intermediate CCI was prepared following Method M from Intermediate CLXXIV (103 mg, 1 eq), 3-hydroxyphenylboronic acid (45 mg, 1.5 eq), PdCl₂(PPh₃)₂ (23 mg, 0.15 eq) and Na₂CO₃ 2M (0.32 mL, 3 eq) in dioxane (2 mL) at 100 ºC for 1 h. Purification by chromatography in silica gel (0% to 40% EtOAc in c-Hex) gave 45 mg, 40%.
HPLC-MS (method 4): Rt = 4.5 min, [M+H]+ 490.1.

### Synthesis of Intermediate CCIV

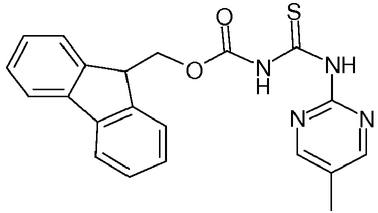

A mixture of Fmoc-Isothiocyanate (0.5 g, 1 eq) and 5-methylpyrimidin-2-amine (194 mg, 1 eq) in toluene (9 mL) was heated at reflux for 90 min. On cooling, the solid was filtered and rinsed with toluene rendering Intermediate CCIV (615 mg, 89%).

### Synthesis of Intermediate CCV

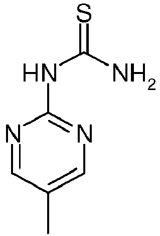

Intermediate CCIV (615 mg, 1 eq) was stirred at rt in a mixture of DCM:piperidine 5:1 (18 mL) for 3 days. The solid was filtered off and triturated with water to afford Intermediate CCV (245 mg, 93%, white solid).
HPLC-MS (method 4): Rt = 1.5 min [M+H]+ 169.1.
¹H NMR (300 MHz, DMSO) δ 10.50 (s, 1H), 10.17 (s, 1H), 9.07 (s, 1H), 8.50 (s, 2H), 2.21 (s, 3H).

### Synthesis of Intermediate CCVI

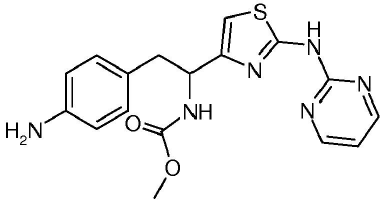

Intermediate CCVI was prepared by Boc-deprotection of Intermediate CXXXIII (30 mg, 1 eq) following Method J. The resulting HCI salt was treated with 7N NH3 in MeOH (3 mL) at rt for 1 h and concentrated. The residue was purified by chromatography (0% to 1% MeOH in DCM) to yield 15 mg, 63%, yellow solid.
HPLC-MS (method 1): Rt = 2.4 min, [M+H]+ 371.0.
¹H NMR (300 MHz, DMSO) δ 11.69 (s, 1H), 8.62 (d, *J* = 4.8 Hz, 2H), 7.47 (d, *J* = 8.8 Hz, 1H), 7.01 (t, *J* = 4.8 Hz, 1H), 6.84 (d, *J* = 8.1 Hz, 2H), 6.71 (s, 1H), 6.44 (d, *J* = 8.3 Hz, 2H), 4.85 (s, 2H), 4.65 (m, 1H), 3.46 (s, 3H), 3.01 (m, 1H), 2.67 (m, 1H).

### Synthesis of final products

The specific process of synthesis of some compounds of the present invention, and their structural formulae, are described below. As it has been commented above, the configuration of the compounds in the formulae is randomly assigned and it represents indeed the order of elution in the HPLC process of separation: first elution peak is drawn with up wedge bond and the second elution peak is drawn with down wedge bond. In order to facilitate the references and denominations of each enantiomer, in some points of the present application, the compound which corresponds to the first elution peak (up wedge bond in the structural formulae) has been named the S-enantiomer, while the compound which corresponds to the second elution peak (down wedge bond in the structural formulae) has been named the R-enantiomer.

### 1.1. Synthesis of Final Compound 1

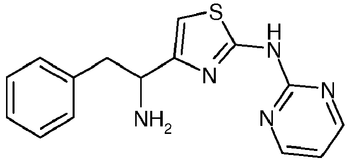

Compound **1** was synthesized following Method E by reaction of Intermediate IV (2.07 g, 1 eq) and hydrazine hydrate (7 mL, 15 eq) in EtOH (95 mL). The compound was purified in silica gel (Biotage, C-40g, 0% to 10% MeOH in DCM) to give the Final Compound **1** (1.3 g, white solid, 90% yield).
HPLC-MS (method 1): Rt = 2.6 min [M+H]+ 281.2 (fragmentation).
1H NMR (300 MHz, DMSO) δ 8.61 (d, *J* = 4.8 Hz, 2H), 7.30 - 7.20 (m, 2H), 7.19-7.08 (m, 3H), 7.01 (t, *J* = 4.8 Hz, 1H), 6.73 (d, *J* = 0.6 Hz, 1H), 4.05 (dd, *J* = 7.5, 5.6 Hz, 1H), 3.11 (dd, *J* = 13.2, 5.4 Hz, 1H), 2.82 - 2.73 (m, 1H).

### 1.2. Synthesis of Final Compound 2

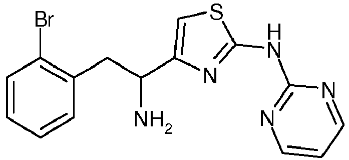

Compound **2** was synthesized following Method E by reaction of Intermediate VIII (1.07 g, 1 eq) and hydrazine hydrate (3 mL, 15 eq) in EtOH (40 mL). The compound was purified in silica gel (Biotage, C-40g, 0% to 10% MeOH in DCM) to give the Final Compound **2** (420 mg, white solid, 52% yield).
HPLC-MS (method 1): Rt = 3.0 min [M+H]+ 359.0/361.0 (fragmentation).
1H NMR (300 MHz, DMSO) δ 8.61 (d, *J* = 4.8 Hz, 2H), 7.57 (m, 1H), 7.25 (m, 2H), 7.12 (m, 1H), 7.01 (t, *J* = 4.8 Hz, 1H), 6.76 (d, *J* = 0.4 Hz, 1H), 4.10 (m, 1H), 3.20 (m, 1H), 2.90 (m, 1H).

### 1.3. Synthesis of Final Compound 3

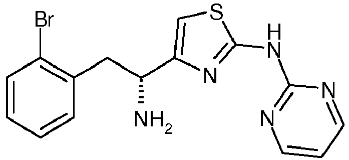

Compound **3** was purified from Final Compound **2** following Method F. Chiral column: Chiral IE; Heptane/EtOH/EDA 70:30:0.1; 5 mL/min; 90 min; 270 nm; Rt= 70 min. The product is isolated contaminated with salts of EDA and it was purified by chromatography in silica gel (5% to 10% MeOH in DCM) to render final compound **3** (3 mg, white solid, ee > 99%).
HPLC-MS (method 1): Rt = 3.0 min [M+H]+ 359.0/361.0 (fragmentation).
1H NMR (300 MHz, DMSO) δ 8.62 (d, *J* = 4.8 Hz, 2H), 7.57 (m, 1H), 7.26 (m, 2H), 7.12 (m, 1H), 7.01 (t, *J* = 4.8 Hz, 1H), 6.77 (d, *J* = 0.4 Hz, 1H), 4.10 (m, 1H), 3.20 (m, 1H), 2.91 (m, 1H).

### 1.4. Synthesis of Final Compound 4

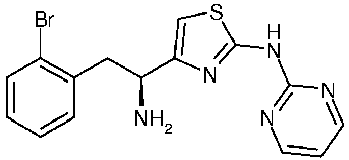

Compound **4** was purified from Final Compound **2** following Method F. Chiral column: Chiral IE; Heptane/EtOH/EDA 70:30:0.1; 5 mL/min; 90 min; 270 nm; Rt= 53 min. The product is isolated contaminated with salts of EDA and it was purified by chromatography in silica gel (5% to 10% MeOH in DCM) to render Final Compound **4** (4 mg, white solid, ee > 99%).
HPLC-MS (method 1): Rt = 3.0 min [M+H]+ 359.0/361.0 (fragmentation).
1H NMR (300 MHz, DMSO) δ 8.61 (d, *J* = 4.8 Hz, 2H), 7.57 (m, 1H), 7.25 (m, 2H), 7.12 (m, 1H), 7.01 (t, *J* = 4.8 Hz, 1H), 6.76 (d, *J* = 0.4 Hz, 1H), 4.10 (m, 1H), 3.20 (m, 1H), 2.90 (m, 1H).

### 1.5. Synthesis of Final Compound 5

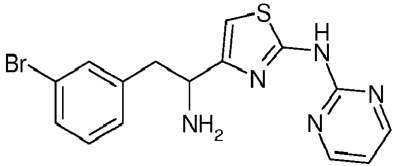

Compound **5** was synthesized following Method E by reaction of Intermediate XII (1.23 g, 1 eq) and hydrazine hydrate (3.5 mL, 15 eq) in EtOH (45 mL). The compound was purified in silica gel (Biotage, C-40g, 0% to 10% MeOH in DCM) to give the Final Compound **5** (650 mg, white solid, 71% yield).
HPLC-MS (method 1): Rt = 3.3 min [M+H]+ 359.0/361.0 (fragmentation).
1H NMR (300 MHz, DMSO) δ 8.61 (d, *J* = 4.8 Hz, 2H), 7.36 (m, 2H), 7.19 (m, 1H), 7.11 (d, *J* = 7.7 Hz, 1H), 7.01 (t, *J* = 4.8 Hz, 1H), 6.74 (d, *J* = 0.7 Hz, 1H), 4.03 (dd, *J* = 7.4, 5.5 Hz, 1H), 3.08 (dd, *J* = 13.2, 5.3 Hz, 1H), 2.80 (dd, *J* = 13.3, 7.9 Hz, 1H).

### 1.6. Synthesis of Final Compound 6

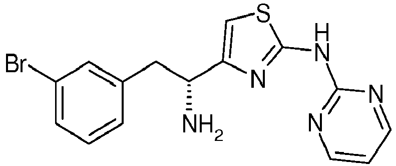

Compound **6** was purified from Final Compound **5** following Method F. Chiral column: Chiral IE; Heptane/EtOH/EDA 70:30:0.1; 5 mL/min; 85 min; 300 nm; Rt= 66 min. The product is isolated contaminated with salts of EDA and it was purified by chromatography in silica gel (5% to 10% MeOH in DCM) to render Final Compound 6 (10 mg, white solid, ee > 99%).
HPLC-MS (method 1): Rt = 3.3 min [M+H]+ 359.0/361.0 (fragmentation).
1H NMR (300 MHz, DMSO) δ 8.61 (d, *J* = 4.8 Hz, 2H), 7.36 (m, 2H), 7.20 (t, *J* = 7.9 Hz, 1H), 7.12 (d, *J* = 7.7 Hz, 1H), 7.01 (t, *J* = 4.8 Hz, 1H), 6.73 (d, *J* = 0.6 Hz, 1H), 4.02 (dd, *J* = 7.4, 5.4 Hz, 1H), 3.08 (dd, *J* = 13.2, 5.2 Hz, 1H), 2.80 (dd, *J* = 13.3, 7.9 Hz, 1H).

### 1.7. Synthesis of Final Compound 7

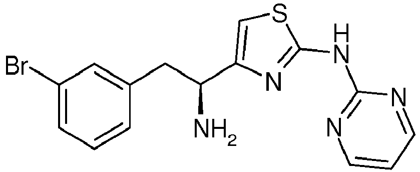

Compound **7** was purified from Final Compound **5** following Method F. Chiral column: Chiral IE; Heptane/EtOH/EDA 70:30:0.1; 5 mL/min; 85 min; 300 nm; Rt= 40 min. The product is isolated contaminated with salts of EDA and it was purified by chromatography in silica gel (5% to 10% MeOH in DCM) to render Final Compound 7 (11 mg, white solid, ee > 99%).
HPLC-MS (method 1): Rt = 3.3 min [M+H]+ 359.0/361.0 (fragmentation).
1H NMR (300 MHz, DMSO) δ 8.62 (d, *J* = 4.8 Hz, 2H), 7.36 (m, 2H), 7.20 (m, 1H), 7.12 (d, *J* = 7.7 Hz, 1H), 7.01 (t, *J* = 4.8 Hz, 1H), 6.74 (d, *J* = 0.6 Hz, 1H), 4.04 (dd, *J* = 7.3, 5.6 Hz, 1H), 3.08 (dd, *J* = 13.2, 5.4 Hz, 1H), 2.81 (dd, *J* = 13.2, 7.8 Hz, 1H).

### 1.8. Synthesis of Final Compound 8

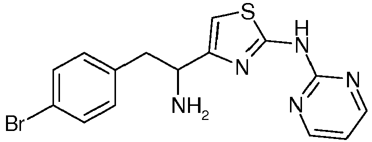

Compound **8** was synthesized following Method E by reaction of Intermediate XVI (1.38 g, 1 eq) and hydrazine hydrate (4 mL, 15 eq) in EtOH (50 mL). The compound was purified in silica gel (Biotage, C-40g, 0% to 10% MeOH in DCM) to give the Final Compound **8** (540 mg, white solid, 52% yield).
HPLC-MS (method 1): Rt = 3.0 min [M+H]+ 359.0/361.0 (fragmentation).
1H NMR (300 MHz, DMSO) δ 8.61 (d, *J* = 4.8 Hz, 2H), 7.41 (d, *J* = 8.4 Hz, 2H), 7.07 (d, *J* = 8.4 Hz, 2H), 7.01 (t, *J* = 4.8 Hz, 1H), 6.70 (d, *J* = 0.7 Hz, 1H), 4.01 (dd, *J* = 7.1, 5.7 Hz, 1H), 3.05 (dd, *J* = 13.2, 5.4 Hz, 1H), 2.78 (m, 1H).

### 1.9. Synthesis of Final Compound 12

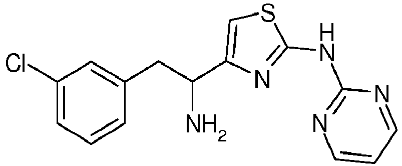

Compound **12** was synthesized following Method E by reaction of Intermediate XXIV (225 mg, 1 eq) and hydrazine hydrate (0.3 mL, 5 eq) in EtOH (10 mL). The compound was purified in silica gel (0% to 15% MeOH in EtOAc) to give the Final Compound **12** (95 mg, white solid, 59% yield).
HPLC-MS (method 1): Rt = 2.9 min [M+H]+ 315.1 (fragmentation).
1H NMR (300 MHz, DMSO) δ 8.62 (d, *J* = 4.8 Hz, 2H), 7.31 - 7.18 (m, 3H), 7.08 (dd, *J* = 7.0, 1.6 Hz, 1H), 7.01 (t, *J* = 4.8 Hz, 1H), 6.73 (d, *J* = 0.6 Hz, 1H), 4.03 (dd, *J* = 7.4, 5.4 Hz, 1H), 3.09 (dd, *J* = 13.2, 5.3 Hz, 1H), 2.81 (dd, *J* = 13.3, 7.9 Hz, 1H).

### 1.10. Synthesis of Final Compound 14

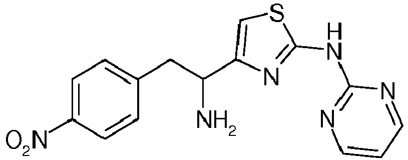

Compound **14** was synthesized following Method E by reaction of Intermediate XL (50 mg, 1 eq) and hydrazine hydrate (0.15 mL, 15 eq) in EtOH (2 mL). The compound was purified in silica gel (Biotage, C-12g, 0% to 15% MeOH in DCM) to give the Final Compound 14 (15 mg, white solid, 41% yield).
HPLC-MS (method 1): Rt = 2.5 min [M+H]+ 326.1 (fragmentation).
1H NMR (300 MHz, DMSO) δ 8.61 (d, *J* = 4.8 Hz, 2H), 8.11 (d, *J* = 8.7 Hz, 2H), 7.40 (d, *J* = 8.7 Hz, 2H), 7.01 (t, *J* = 4.8 Hz, 1H), 6.73 (s, 1H), 4.09 (m, 1H), 3.21 (dd, *J* = 13.0, 5.5 Hz, 1H), 2.97 (dd, *J* = 13.1, 7.7 Hz, 1H).

### 1.11. Synthesis of Final Compound 17

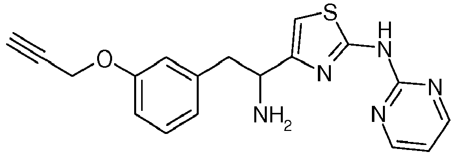

Compound **17** was synthesized following Method E by reaction of Intermediate LII (490 mg, 1 eq) and methylhydrazine (1 mL, 20 eq) in EtOH (20 mL). The compound was purified in silica gel (0% to 10% MeOH in DCM) to give the Final Compound **17** (50 mg, white solid, 14% yield).
HPLC-MS (method 1): Rt = 3.0 min [M+H]+ 335.1 (fragmentation).
1H NMR (300 MHz, DMSO) δ 8.62 (d, *J* = 4.8 Hz, 2H), 7.18 (t, *J* = 7.8 Hz, 1H), 7.02 (t, *J* = 4.8 Hz, 1H), 6.77 (m, 4H), 4.73 (d, *J* = 2.3 Hz, 2H), 4.11 (m, 1H), 3.54 (t, *J* = 2.3 Hz, 1H), 3.11 (dd, *J* = 13.1, 5.7 Hz, 1H), 2.79 (dd, *J* = 13.0, 7.8 Hz, 1H).

### 1.12. Synthesis of Final Compound 18

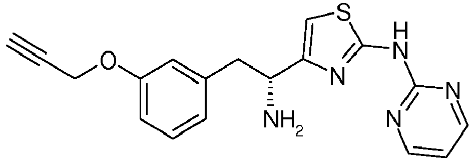

Compound **18** was purified from Final Compound **17** following Method F. Chiral column: Chiral IE; Heptane/EtOH/EDA 70:30:0.1; 5 mL/min; 95 min; 290 nm; Rt= 70 min. The product is isolated contaminated with salts of EDA and it was purified by chromatography in silica gel (5% MeOH in DCM) to render Final Compound **18** (10 mg, white solid, ee > 99%).
HPLC-MS (method 1): Rt = 3.0 min [M+H]+ 335.1 (fragmentation).
1H NMR (300 MHz, DMSO) δ 8.61 (d, *J* = 4.8 Hz, 2H), 7.17 (t, *J* = 7.8 Hz, 1H), 7.01 (t, *J* = 4.8 Hz, 1H), 6.77 (m, 4H), 4.73 (d, *J* = 2.4 Hz, 2H), 4.07 (dd, *J* = 7.7, 5.5 Hz, 1H), 3.53 (m, 1H), 3.10 (dd, *J* = 13.2, 5.5 Hz, 1H), 2.76 (dd, *J* = 11.7, 6.3 Hz, 1H).

### 1.13. Synthesis of Final Compound 19

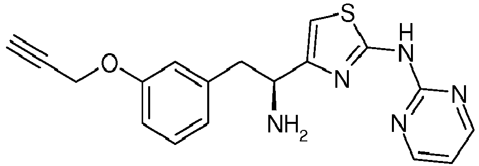

Compound **19** was purified from Final Compound **17** following Method F. Chiral column: Chiral IE; Heptane/EtOH/EDA 70:30:0.1; 5 mL/min; 95 min; 290 nm; Rt= 39 min. The product is isolated contaminated with salts of EDA and it was purified by chromatography in silica gel (5% MeOH in DCM) to render Final Compound **19** (10 mg, white solid, ee > 99%).
HPLC-MS (method 1): Rt = 3.0 min [M+H]+ 335.1 (fragmentation).
1H NMR (300 MHz, DMSO) δ 8.61 (d, *J* = 4.8 Hz, 2H), 7.17 (t, *J* = 7.8 Hz, 1H), 7.01 (t, *J* = 4.8 Hz, 1H), 6.78 (m, 4H), 4.73 (d, *J =* 2.4 Hz, 2H), 4.07 (dd, *J* = 7.7, 5.5 Hz, 1H), 3.54 (m, 1H), 3.10 (dd, *J* = 13.2, 5.5 Hz, 1H), 2.76 (dd, *J* = 11.7, 6.3 Hz, 1H).

### 1.14. Synthesis of Final Compound 20

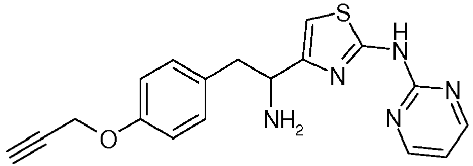

Compound **20** was synthesized following Method E by reaction of Intermediate LVI (90 mg, 1 eq) and methylhydrazine (0.2 mL, 20 eq) in EtOH (4 mL). The compound was purified in silica gel (0% to 5% MeOH in DCM) to give Final Compound **20** (23 mg, white solid, 35% yield).
HPLC-MS (method 1): Rt = 2.9 min [M+H]+ 335.1 (fragmentation).
1H NMR (300 MHz, DMSO) δ 8.61 (d, *J* = 4.8 Hz, 2H), 7.06 (d, *J* = 8.6 Hz, 2H), 7.01 (t, *J* = 4.8 Hz, 1H), 6.86 (d, *J* = 8.5 Hz, 2H), 6.73 (s, 1H), 4.73 (d, *J* = 2.3 Hz, 2H), 4.00 (dd, *J* = 7.5, 5.6 Hz, 1H), 3.53 (dd, *J* = 7.6, 5.4 Hz, 1H), 3.05 (dd, *J* = 13.3, 5.3 Hz, 1H), 2.73 (m, 1H).

### 1.15. Synthesis of Final Compound 21

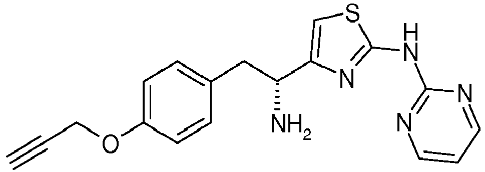

Compound **21** was purified from Final Compound **20** following Method F. Chiral column: Chiral IE; Heptane/EtOH/EDA 70:30:0.1; 7 mL/min; 60 min; 280 nm; Rt= 41 min. The product is isolated contaminated with salts of EDA and it was purified by chromatography in silica gel (5% MeOH in DCM) to render Final Compound **21** (36 mg, white solid, ee > 99%).
HPLC-MS (method 1): Rt = 2.9 min [M+H]+ 335.1 (fragmentation).
1H NMR (300 MHz, DMSO) δ 8.62 (d, *J* = *4.8* Hz, 2H), 7.06 (d, *J* = 8.7 Hz, 2H), 7.01 (t, *J* = 4.8 Hz, 1H), 6.86 (d, *J* = 8.7 Hz, 2H), 6.74 (s, 1H), 4.73 (d, *J* = 2.4 Hz, 2H), 4.08 - 3.97 (m, 1H), 3.55 (t, *J* = 2.4 Hz, 1H), 3.05 (dd, *J* = 13.2, 5.4 Hz, 1H), 2.79 - 2.74 (m, 1H).

### 1.16. Synthesis of Final Compound 22

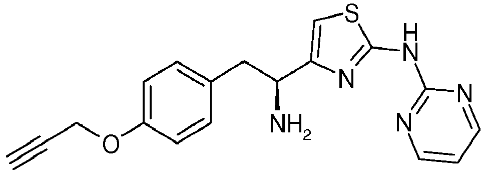

Compound **22** was purified from Final Compound **20** following Method F. Chiral column: Chiral IE; Heptane/EtOH/EDA 70:30:0.1; 7 mL/min; 60 min; 280 nm; Rt= 30 min. The product is isolated contaminated with salts of EDA and it was purified by chromatography in silica gel (5% MeOH in DCM) to render Final Compound **22** (37 mg, white solid, ee > 99%).
HPLC-MS (method 1): Rt = 2.9 min [M+H]+ 335.1 (fragmentation).
1H NMR (300 MHz, DMSO) δ 8.61 (d, *J* = 4.8 Hz, 2H), 7.05 (d, *J* = 8.7 Hz, 2H), 7.00 (t, *J* = 4.8 Hz, 1H), 6.85 (d, *J* = 8.7 Hz, 2H), 6.72 (s, 1H), 4.72 (d, *J* = 2.4 Hz, 2H), 4.05 - 3.96 (m, 1H), 3.54 (t, *J* = 2.4 Hz, 1H), 3.04 (dd, *J* = 13.4, 5.7 Hz, 1H), 2.73 (dd, *J* = 7.2, 5.1 Hz, 1H).

### 1.17. Synthesis of Final Compound 26

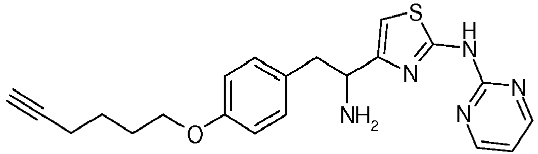

Compound **26** was synthesized following Method E by reaction of Intermediate LXIV (340 mg, 1 eq) and methylhydrazine (0.7 mL, 20 eq) in EtOH (13 mL). The compound was purified in silica gel (Biotage, C-20g, 0% to 10% MeOH in DCM) to give Final Compound **26** (90 mg, white solid, 35% yield).
HPLC-MS (method 1): Rt = 3.4 min [M+H]+ 377.1 (fragmentation).
1H NMR (300 MHz, DMSO) δ 8.62 (d, *J* = 4.8 Hz, 2H), 7.01 (m, 3H), 6.78 (m, 3H), 4.05 (t, *J* = 6.6 Hz, 1H), 3.91 (t, *J* = 6.3 Hz, 2H), 3.05 (dd, *J* = 13.3, 5.8 Hz, 1H), 2.77 (m, 2H), 2.21 (m, 2H), 1.76 (m, 2H), 1.57 (m, 2H).

### 1.18. Synthesis of Final Compound 27

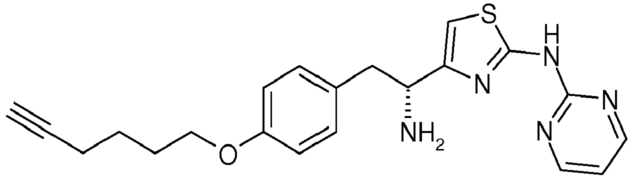

Compound **27** was purified from Final Compound **26** following Method F. Chiral column: Chiral IE; Heptane/EtOH/EDA 70:30:0.1; 5 mL/min; 90 min; 300 nm; Rt= 63 min. The product is isolated contaminated with salts of EDA and it was purified by chromatography in silica gel (5% MeOH in DCM) to render Final Compound **27** (20 mg, white solid, ee > 99%).
HPLC-MS (method 1): Rt = 3.4 min [M+H]+ 377.1 (fragmentation).
1H NMR (300 MHz, DMSO) δ 8.62 (d, *J* = 4.8 Hz, 2H), 7.02 (m, 3H), 6.80 (d, *J* = 8.7 Hz, 2H), 6.73 (d, *J* = 0.5 Hz, 1H), 4.02 (m, 1H), 3.92 (t, *J* = 6.3 Hz, 2H), 3.04 (dd, *J* = 13.3, 5.5 Hz, 1H), 2.76 (m, 2H), 2.24 (m, 2H), 1.77 (m, 2H), 1.58 (m, 2H).

### 1.19. Synthesis of Final Compound 28

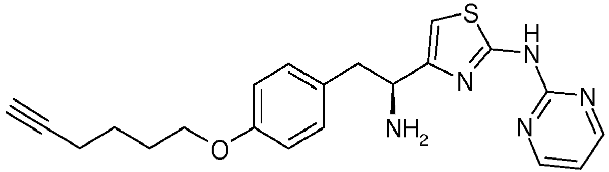

Compound **28** was purified from Final Compound **26** following Method F. Chiral column: Chiral IE; Heptane/EtOH/EDA 70:30:0.1; 5 mL/min; 90 min; 300 nm; Rt= 44 min. The product is isolated contaminated with salts of EDA and it was purified by chromatography in silica gel (5% MeOH in DCM) to render Final Compound **28** (24 mg, white solid, ee > 99%).
HPLC-MS (method 1): Rt = 3.4 min [M+H]+ 377.1 (fragmentation).
1H NMR (300 MHz, DMSO) δ 8.61 (d, *J* = 4.8 Hz, 2H), 7.02 (m, 3H), 6.79 (d, *J* = 8.7 Hz, 2H), 6.73 (d, *J* = 0.5 Hz, 1H), 4.01 (m, 1H), 3.92 (t, *J* = 6.3 Hz, 2H), 3.04 (dd, *J* = 13.3, 5.5 Hz, 1H), 2.75 (m, 2H), 2.23 (m, 2H), 1.77 (m, 2H), 1.57 (m, 2H).

### 1.20. Synthesis of Final Compound 33

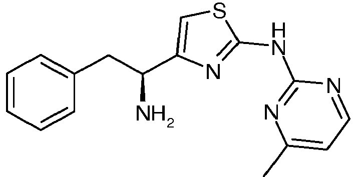

Compound **33** was synthesized following Method J by reaction of Intermediate CXII (83 mg, 1 eq) in dioxane (2 mL) and HCI 4N in dioxane (0.25 mL, 25 eq) to give Final Compound **33** after isolation with SCX-2 as a free base (58 mg, white solid, 92%). HPLC-MS (method 1): Rt = 2.8 min, [M+H]+ 295.0 (fragmentation).
1H NMR (300 MHz, DMSO) δ□8.47 (d, *J* = 5.0 Hz, 1H), 7.29-7.16 (m, 3H), 7.15-7.08 (m, 2H), 6.92 (d, J = 5.1 Hz, 1H), 6.84 (s, 1H), 4.25 (t, *J* = 6.9 Hz, 1H), 3.17 (dd, *J* = 13.3, 7.2 Hz, 1H), 2.97 (dd, *J* = 13.4, 6.7 Hz, 1H), 2.43 (s, 3H).

### 1.21. Synthesis of Final Compound 34

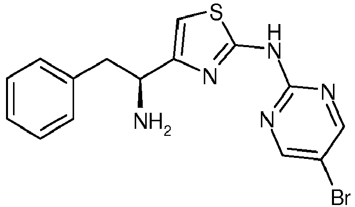

Compound **34** was synthesized following Method N by reaction of Intermediate CXIII (50 mg, 1 eq) and TFA (0.16 mL, 20 eq) to give Final Compound **34** after isolation with SCX-2 as a free base (22 mg, white solid, 55%).
HPLC-MS (method 1): Rt = 3.17 min, [M+H]+ 359/361.1 (fragmentation).
1H NMR (300 MHz, DMSO) δ 8.76 (s, 2H), 7.30-7.10 (m, 5H), 6.78 (d, *J* = 0.6 Hz, 1H), 4.05 (dd, *J* = 7.5, 5.5 Hz, 1H), 3.11 (dd, *J* = 13.2, 5.4 Hz, 1H), 2.84 - 2.73 (m, 1H).

### 1.22. Synthesis of Final Compound 35

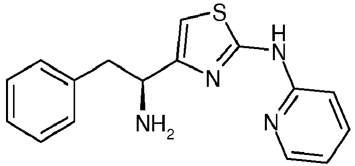

Compound **35** was synthesized following Method J by reaction of Intermediate CXIV (125 mg, 1 eq) in dioxane (3 mL) and HCl 4N in dioxane (1.18 mL, 15 eq) to give Final Compound **35** after isolation with SCX-2 as a free base (528 mg, white solid, 56%). HPLC-MS (method 1): Rt = 2.4 min, [M+H]+ 280 (fragmentation).
1H NMR (300 MHz, DMSO) δ 11.28 (s, 1H), 8.30 - 8.20 (m, 1H), 7.75 - 7.63 (m, 1H), 7.29 - 7.16 (m, 3H), 7.15 - 7.10 (m, 2H), 7.04 (d, *J* = 8.4 Hz, 1H), 6.90 (ddd, *J* = 7.2, 5.1, 0.9 Hz, 1H), 4.11 - 4.03 (m, 1H), 3.12 (dd, *J* = 13.2, 5.8 Hz, 1H), 2.82 (dd, *J* = 13.2, 7.6 Hz, 1H).

### 1.23. Synthesis of Final Compound 36

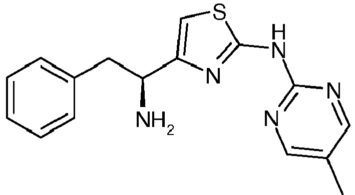

Compound **36** was synthesized following Method N by reaction of Intermediate CXV (85 mg, 1 eq) and TFA (0.3 mL, 20 eq) to give Final Compound **36** after isolation with SCX-2 as a free base (41 mg, white solid, 63%).
HPLC-MS (method 1): Rt = 2.9 min, [M+H]+ 295.0 (fragmentation).
1H NMR (300 MHz, DMSO) δ 8.47 (s, 2H), 7.25 (t, *J* = 7.1 Hz, 2H), 7.20-7.10 (m, 3H), 6.69 (s, 1H), 4.03 (dd, *J* = 7.5, 5.4 Hz, 1H), 3.11 (dd, *J* = 13.2, 5.2 Hz, 1H), 2.76 (dd, *J* = 13.2, 8.0 Hz, 1H), 2.20 (s, 3H).

### 1.24. Synthesis of Final Compound 37

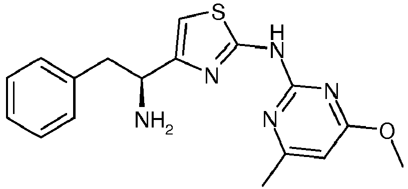

Compound **37** was synthesized following Method J by reaction of Intermediate CXVI (65 mg, 1 eq) in dioxane (3 mL) and HCI 4N in dioxane (0.75 mL, 20 eq) to give Final Compound **37** after isolation with SCX-2 as a free base (14 mg, white solid, 28%). HPLC-MS (method 1): Rt = 3.0 min, [M+H]+ 342.0 and 325.0 (fragmentation).
1H NMR (300 MHz, DMSO) δ 7.30 - 7.10 (m, 5H), 6.77 (s, 1H), 6.33 (s, 1H), 4.18-4.07 (m, 1H), 3.98 (s, 3H), 3.13 (dd, *J* = 13.1, 5.9 Hz, 1H), 2.85 (dd, *J* = 12.9, 7.5 Hz, 1H), 2.33 (s, 3H).

### 1.25. Synthesis of Final Compound 52

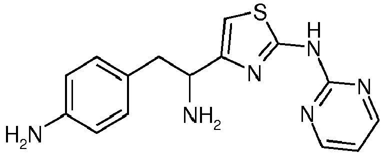

A solution of Intermediate CCVI (5 mg, 1 eq) and LiOH.H₂O (8 mg, 15 eq) in MeOH:*ⁱ*PrOH (1:1, 1 mL) was heated under microwave irradiation at 160 ºC for 1 h. On cooling, the mixture was concentrated and the residue was purified by chromatography (1% to 7% MeOH in DCM) to give Final Compound **52** (2 mg, off-white solid, 47%).
HPLC-MS (method 3): Rt = 2.6 min, [M+H]+ 296.1 (fragmentation).
1H NMR (300 MHz, DMSO) δ 8.62 (d, *J* = 4.8 Hz, 2H), 7.01 (t, *J* = 4.8 Hz, 1H), 6.78 (m, 3H), 6.44 (d, *J* = 8.3 Hz, 2H), 4.85 (bs, 2H), 4.02 (t, *J* = 6.5 Hz, 1H), 2.96 (dd, *J* = 13.3, 5.9 Hz, 1H), 2.65 (dd, *J* = 13.4, 7.5 Hz, 1H).

### 1.26. Synthesis of Final Compound 58

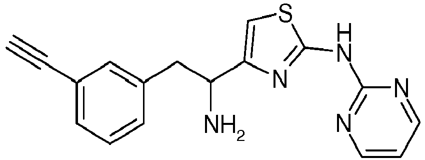

Compound **58** was synthesized following Method S by reaction of Intermediate CXLI (63 mg, 1 eq) with Amberlyst®-15. The compound was purified by chromatography (2% to 7% MeOH in DCM) to yield Final Compound **58** (6 mg, 14%, white solid).
HPLC-MS (method 1): Rt = 2.9 min, [M+H]+ 305.1 (fragmentation).
1H NMR (300 MHz, DMSO) δ 8.62 (d, *J* = 4.8 Hz, 2H), 7.27 (m, 3H), 7.16 (m, 1H), 7.01 (t, *J* = 4.8 Hz, 1H), 6.75 (s, 1H), 4.12 (s, 1H), 4.07 (dd, *J* = 7.6, 5.7 Hz, 1H), 3.10 (dd, *J* = 13.3, 5.6 Hz, 1H), 2.82 (dd, *J* = 13.3, 7.7 Hz, 1H).

### 1.27. Synthesis of Final Compound 60

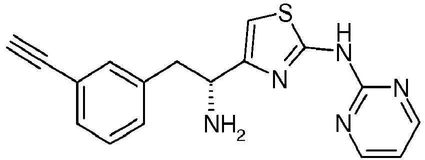

Chiral separation of racemate mixture Compound **58** following Method F. Chiral column: Chiral IE; Heptane/EtOH/EDA 70:30:0.1; 6 mL/min; 85 min; 280 nm; Rt= 63 min. Purification then by silica gel (5% to 10% MeOH in DCM) yielded Final compound 60 (17 mg, white solid, ee > 99 %).
HPLC-MS (method 1): Rt = 2.9 min, [M+H]+ 305.1 (fragmentation).
1H NMR (300 MHz, DMSO) δ 8.62 (d, *J* = 4.8 Hz, 2H), 7.33 - 7.21 (m, 3H), 7.21 - 7.11 (m, 1H), 7.01 (t, *J* = 4.8 Hz, 1H), 6.74 (d, *J* = 0.6 Hz, 1H), 4.13 (s, 1H), 4.04 (dd, *J* = 7.6, 5.5 Hz, 1H), 3.09 (dd, *J* = 13.2, 5.3 Hz, 1H), 2.87 - 2.74 (m, 1H).

### 1.28. Synthesis of Final Compound 61

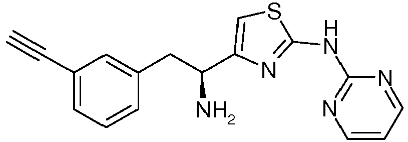

Chiral separation of racemate mixture Compound **58** following Method F. Chiral column: Chiral IE; Heptane/EtOH/EDA 70:30:0.1; 6 mL/min; 85 min; 280 nm; Rt= 34 min. Purification then by silica gel (5% to 10% MeOH in DCM) gave Final Compound **61** (16 mg, white solid, ee > 99 %).
HPLC-MS (method 1): Rt = 2.9 min, [M+H]+ 305.1 (fragmentation).
1H NMR (300 MHz, DMSO) δ 8.62 (d, *J* = 4.8 Hz, 2H), 7.34 - 7.20 (m, 3H), 7.20 - 7.10 (m, 1H), 7.01 (t, *J* = 4.8 Hz, 1H), 6.74 (s, 1H), 4.13 (s, 1H), 4.04 (dd, *J* = 7.6, 5.5 Hz, 1H), 3.09 (dd, *J* = 13.2, 5.3 Hz, 1H), 2.80 (dd, *J* = 13.2, 7.8 Hz, 1H).

### 1.29. Synthesis of Final Compound 62

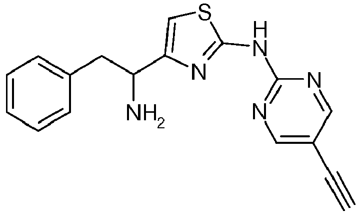

Compound **62** was synthesized following Method S by reaction of Intermediate CXLIII (63 mg, 1 eq) and Amberlyst®-15. Traces of TMS protecting group were detected, so the compound was suspended in MeOH (5 mL) and K₂CO₃ (50 mg) was added stirring for 2.5 h. The mixture was filtered, concentrated and purified by SCX-2 cartridge to yield Final Compound **62** (40 mg, 56%, off-white solid).
HPLC-MS (method 1): Rt = 3.1 min, [M+H]+ 305.0 (fragmentation).
1H NMR (300 MHz, DMSO) δ 8.74 (s, 2H), 7.23 (m, 2H), 7.14 (m, 3H), 6.78 (s, 1H), 4.47 (s, 1H), 4.05 (m, 1H), 3.10 (dd, *J* = 13.1, 5.5 Hz, 1H), 2.78 (dd, *J* = 13.2, 7.9 Hz, 1H).

### 1.30. Synthesis of Final Compound 63

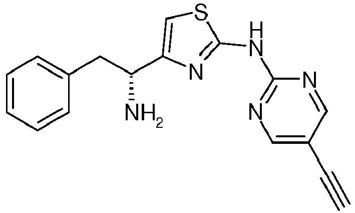

Chiral separation of racemate mixture Compound **62** following Method F. Chiral column: Chiral IE; Heptane/EtOH/EDA 70:30:0.1; 5 mL/min; 60 min; 310 nm; Rt= 43 min. Purification then by chromatography in silica gel (5% MeOH in DCM) gave Final compound **63** (6 mg, white solid, ee > 99 %).
HPLC-MS (method 1): Rt = 3.1 min, [M+H]+ 305.1 (fragmentation).
1H NMR (300 MHz, DMSO) δ 8.75 (s, 2H), 7.31 - 7.08 (m, 5H), 6.79 (s, 1H), 4.48 (s, 1H), 4.05 (dd, *J* = 7.7, 5.5 Hz, 1H), 3.11 (dd, *J* = 13.2, 5.3 Hz, 1H), 2.84 - 2.76 (m, 1H).

### 1.31. Synthesis of Final Compound 64

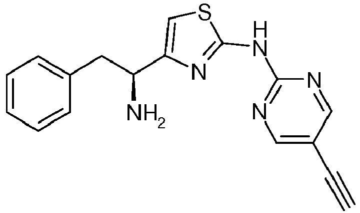

Chiral separation of racemate mixture Compound **62** following Method F. Chiral column: Chiral IE; Heptane/EtOH/EDA 70:30:0.1; 5 mL/min; 60 min; 310 nm; Rt= 33 min. Purification then by chromatography in silica gel (5% MeOH in DCM) gave Final compound **64** (9 mg, white solid, ee > 99 %).
HPLC-MS (method 1): Rt = 3.1 min, [M+H]+ 305.1 (fragmentation).
1H NMR (300 MHz, DMSO) δ 8.75 (s, 2H), 7.33 - 7.09 (m, 5H), 6.80 (s, 1H), 4.48 (s, 1H), 4.07 (dd, *J* = 7.5, 5.6 Hz, 1H), 3.11 (dd, *J* = 13.2, 5.5 Hz, 1H), 2.86 - 2.74 (m, 1H).

### 1.32. Synthesis of Final Compound 66

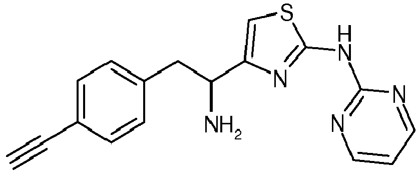

Compound **66** was synthesized following Method S by reaction of Intermediate CXLVI (70 mg, 1 eq) and Amberlyst®-15. The compound was purified by chromatography in silica gel (2% to 7% MeOH in DCM) to yield Final Compound **66** (6 mg, 13%, white solid). HPLC-MS (method 1): Rt = 2.9 min, [M+H]+ 305.0 (fragmentation).
1H NMR (300 MHz, DMSO) δ 8.63 (d, *J* = 4.8 Hz, 2H), 7.36 (d, *J* = 8.1 Hz, 2H), 7.14 (d, *J* = 8.2 Hz, 2H), 7.03 (t, *J* = 4.8 Hz, 1H), 6.75 (s, 1H), 4.10 (m, 2H), 3.12 (dd, *J* = 13.2, 5.8 Hz, 1H), 2.87 (dd, *J* = 13.1, 7.5 Hz, 1H).

### 1.33. Synthesis of Final Compound 68

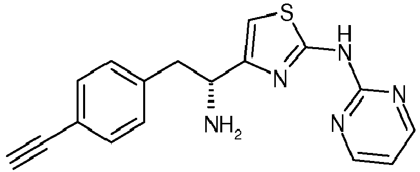

Compound **68** was purified from Final Compound **66** following Method F. Chiral column: Chiral IE; Heptane/EtOH/EDA 70:30:0.1; 5 mL/min; 75 min; 300 nm; Rt= 55 min. The product was isolated contaminated with salts of EDA and was purified by chromatography in silica gel (5% to 10% MeOH in DCM) to render Final Compound **68** (13 mg, white solid, ee > 99%).
HPLC-MS (method 1): Rt = 2.9 min, [M+H]+ 305.0 (fragmentation).
1H NMR (300 MHz, DMSO) δ 8.62 (d, *J* = 4.8 Hz, 2H), 7.34 (d, *J* = 8.1 Hz, 2H), 7.13 (d, *J* = 8.2 Hz, 2H), 7.01 (t, *J* = 4.8 Hz, 1H), 6.72 (d, *J* = 0.5 Hz, 1H), 4.12 (s, 1H), 4.09 - 3.98 (m, 1H), 3.10 (dd, *J* = 13.1, 5.6 Hz, 1H), 2.83 (dd, *J* = 13.1, 7.7 Hz, 1H).

### 1.34. Synthesis of Final Compound 69

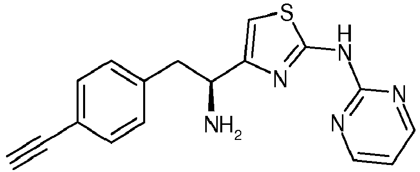

Compound **69** was purified from Final Compound **66** following Method F. Chiral column: Chiral IE; Heptane/EtOH/EDA 70:30:0.1; 5 mL/min; 75 min; 300 nm; Rt= 29 min. The product was isolated contaminated with salts of EDA and was purified by chromatography in silica gel (5% to 10% MeOH in DCM) to render Final Compound **69** (16 mg, white solid, ee > 99%).
HPLC-MS (method 1): Rt = 2.9 min, [M+H]+ 305.0 (fragmentation).
1H NMR (300 MHz, DMSO) δ 8.62 (d, *J* = 4.8 Hz, 2H), 7.34 (d, *J* = 8.2 Hz, 2H), 7.13 (d, *J* = 8.2 Hz, 2H), 7.01 (t, *J* = 4.8 Hz, 1H), 6.72 (d, *J* = 0.6 Hz, 1H), 4.11 (s, 1H), 4.09 - 3.98 (m, 1H), 3.10 (dd, *J* = 13.2, 5.6 Hz, 1H), 2.83 (dd, *J* = 13.2, 7.7 Hz, 1H).

### 1.35. Synthesis of Final Compound 74

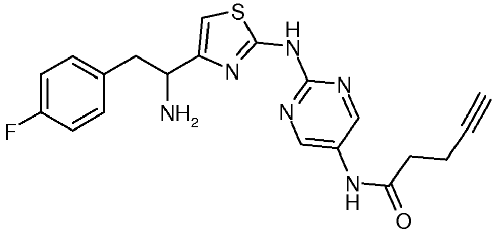

Compound **74** was synthesized following Method E by reaction of Intermediate CLVII (56 mg, 1 eq) with methylhydrazine (0.07 mL, 20 eq) in EtOH (4 mL) and heating at 90 ºC for 3 h. Purification by silica flash chromatography (0% to 20% MeOH in EtOAc) gave Final Compound **74** (14 mg, white solid, 33%).
HPLC-MS (method 1): Rt = 2.90 min, [M+H]+ 411.1.
1H NMR (300 MHz, DMSO) δ 11.60 (s, 1H), 10.26 (s, 1H), 8.79 (s, 2H), 7.10 (m, 4H), 6.75 (s, 1H), 4.11 (t, *J* = 6.6 Hz, 1H), 3.10 (dd, *J* = 13.3, 6.2 Hz, 1H), 2.88 (dd, *J* = 12.5, 6.4 Hz, 1H), 2.83 (t, *J* = 2.5 Hz, 1H), 2.56 (m, 4H, under DMSO signal).

### 1.36. Synthesis of Final Compound 78

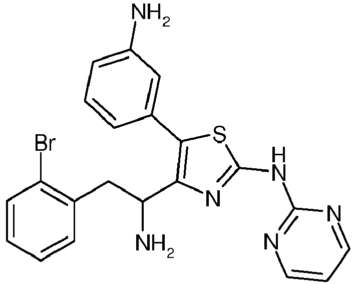

Compound **78** was synthesized following conditions used in Method J by reaction of Intermediate CLXIII (20 mg, 1 eq) in dioxane (1 mL) and HCl 4N (0.1 mL, 10 eq) to give Final Compound **78** after isolation with SCX-2 as a free base and then HPLC preparative (2 mg, off-white solid, 14%).
HPLC-MS (method 1): Rt = 3.0 min, [M+H]+ 450.0 (fragmentation).
1H NMR (300 MHz, DMSO) δ 8.63 (d, *J* = 4.8 Hz, 2H), 7.51 (dd, *J* = 7.8, 1.2 Hz, 1H), 7.17 (dd, *J* = 7.4, 1.3 Hz, 1H), 7.09 (m, 1H), 7.03 (m, 2H), 6.94 (t, *J* = 7.8 Hz, 1H), 6.48 (dd, *J* = 8.0, 1.5 Hz, 1H), 6.37 (s, 1H), 6.19 (d, *J* = 7.7 Hz, 1H), 5.06 (bs, 2H), 4.27 (t, *J* = 7.2 Hz, 1H), 3.14 (m, 2H).

### 1.37. Synthesis of Final Compound 81

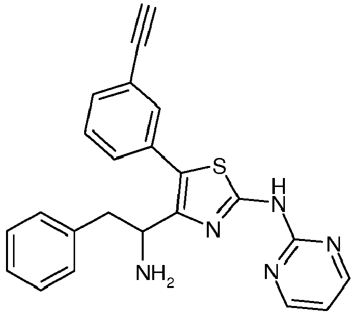

Intermediate CLXIX (83 mg, 1.0 eq) and Amberlyst-(r)-15 (466 mg. 15 eq) were stirred in MeOH (10 mL) at rt for 2 h. The mixture was filtered off and rinsed with methanol. The resin was stirred with NH₃ (7N in MeOH) (5 mL) at rt for 30 min. The resin was filtered and the filtrate was evaporated to render a crude which was still impure with TMS fragment, so the mixture was stirred with K₂CO₃ (15 mg, 1 eq) in MeOH (2 mL) at rt for 1 h. It was concentrated and purified by flash column (0% to 10% MeOH in DCM) to yield Final Compound **81** (15 mg, white solid, 26%).
HPLC-MS (method 1): Rt = 3.5 min, [M+H]+ 381.0 (fragmentation).
¹H NMR (300 MHz, DMSO) δ 8.64 (d, *J* = 4.8 Hz, 2H), 7.41 - 7.28 (m, 2H), 7.21 - 7.01 (m, 6H), 6.94 (dd, *J* = 7.6, 1.8 Hz, 2H), 4.24 (s, 1H), 3.97 (dd, *J* = 8.3, 6.1 Hz, 1H), 3.08 - 3.00 (m, 2H).

### 1.38. Synthesis of Final Compound 83

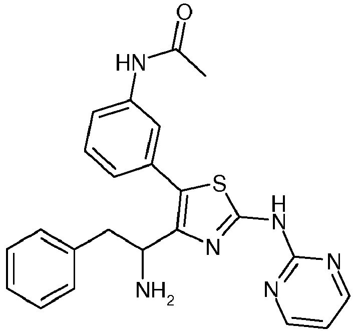

Compound **83** was synthesized following Method E by reaction of Intermediate CLXXIII (70 mg, 1 eq) with hydrazine hydrate 50% (0.052 mL, 15 eq) in EtOH (1 mL) at 90 ºC for 2 h. Purification by silica flash chromatography (0% to 15% MeOH in EtOAc) and then SCX-2 cartridge gave Final Compound **83** (11 mg, white solid, 28%).
HPLC-MS (method 1): Rt = 3.1 min, [M+H]+ 414.0 (fragmentation).
1H NMR (300 MHz, DMSO) δ 9.99 (s, 1H), 8.64 (d, *J* = 4.8 Hz, 2H), 7.58 (s, 1H), 7.48 (d, *J* = 8.1 Hz, 1H), 7.27 - 7.09 (m, 4H), 7.03 (dt, *J* = 6.3, 5.1 Hz, 3H), 6.72 (d, *J* = 7.8 Hz, 1H), 4.08 (t, *J* = 7.1 Hz, 1H), 3.07 (dd, *J* = 12.9, 7.5 Hz, 1H), 2.96 (dd, *J* = 13.0, 7.0 Hz, 1H), 2.05 (s, 3H).

### 1.39. Synthesis of Final Compound 84

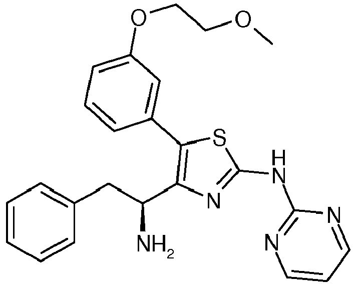

Compound **84** was synthesized following Method N by reaction of Intermediate CLXXV (43 mg, 1 eq) and TFA (0.12 mL, 20 eq) in DCM (2 mL) to give Final Compound **84** after isolation with SCX-2 as a free base (26 mg, white solid, 76%).
HPLC-MS (method 1): Rt = 3.5 min, [M+H]+ 431.1 (fragmentation).
1H NMR (300 MHz, CDCl₃) δ 8.72 (d, *J* = 4.8 Hz, 2H), 7.24 - 7.11 (m, 4H), 6.99 (dd, *J* = 9.4, 3.5 Hz, 2H), 6.94 (t, *J* = 4.9 Hz, 1H), 6.90 - 6.84 (m, 1H), 6.70 (d, *J* = 6.9 Hz, 2H), 4.36 (t, *J* = 7.3 Hz, 1H), 4.06 (dd, *J* = 5.4, 3.9 Hz, 2H), 3.75 (dd, *J* = 5.5, 3.8 Hz, 2H), 3.46 (s, 3H), 3.24 - 3.03 (m, 2H).

### 1.40. Synthesis of Final Compound 87

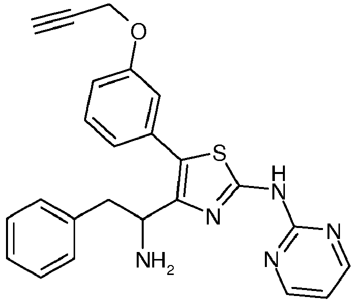

Compound **87** was synthesized following Method E by reaction of Intermediate CLXXIX (72 mg, 1 eq) with methylhydrazine (0.14 mL, 25 eq) in EtOH (2 mL) at 90 ºC for 6 h. Purification by silica flash chromatography (0% to 15% MeOH in EtOAc) and then SCX-2 cartridge gave Final Compound **87** (20 mg, off-white solid, 43%).
HPLC-MS (method 1): Rt = 3.7 min, [M+H]+ 411.1 (fragmentation).
1H NMR (300 MHz, DMSO) δ□8.64 (d, *J* = 4.8 Hz, 2H), 7.29 - 7.21 (m, 1H), 7.18 - 7.09 (m, 3H), 7.03 (t, *J* = 4.8 Hz, 1H), 7.00 - 6.95 (m, 2H), 6.91 (dd, *J* = 7.9, 2.2 Hz, 1H), 6.78 - 6.75 (m, 1H), 6.73 (d, *J* = 7.6 Hz, 1H), 4.76 (d, *J* = 2.4 Hz, 2H), 4.05 (t, *J* = 7.2 Hz, 1H), 3.57 (t, *J* = 2.3 Hz, 1H), 3.07 (dd, *J* = 12.8, 7.9 Hz, 1H), 2.95 (dd, *J=* 12.8, 6.5 Hz, 1H).

### 1.41. Synthesis of Final Compound 88

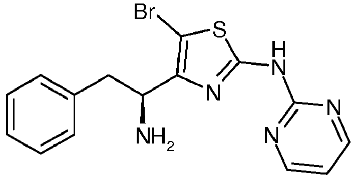

Compound 88 was synthesized following Method N by reaction of Intermediate CLXXIV (60 mg, 1 eq) and TFA (0.23 mL, 20 eq) in DCM (2 mL) to give Final Compound **88** after isolation with SCX-2 as a free base (47 mg, white solid, 99%).
HPLC-MS (method 1): Rt = 3.0 min, [M+H]+ 376.1/378.1.
1H NMR (300 MHz, DMSO) δ 8.64 (d, *J* = 4.8 Hz, 2H), 7.26 - 7.10 (m, 3H), 7.05 (m, 3H), 4.07 (t, *J* = 7.3 Hz, 1H), 3.01 - 2.83 (m, 2H).

### 1.42. Synthesis of Final Compound 89

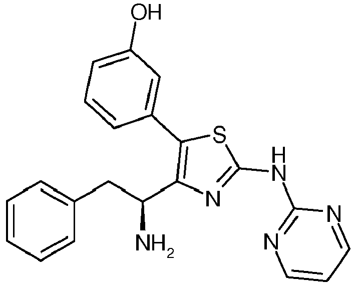

Compound **89** was synthesized following Method N by reaction of Intermediate CCI (43 mg, 1 eq) and TFA (0.17 mL, 25 eq) in DCM (2 mL) to give Final Compound **89** after isolation with SCX-2 as a free base (33 mg, white solid, 96%).
HPLC-MS (method 1): Rt = 3.2 min, [M+H]+ 373.1 (fragmentation).
1H NMR (300 MHz, DMSO) δ 9.52 (s, 1H), 8.64 (d, *J* = 4.8 Hz, 2H), 7.22 - 7.06 (m, 4H), 7.04 (t, *J* = 4.8 Hz, 1H), 7.01 - 6.95 (m, 2H), 6.70 (d, *J* = 1.7 Hz, 1H), 6.62 (s, 1H), 6.45 (d, *J* = 7.8 Hz, 1H), 4.13 (t, *J* = 7.2 Hz, 1H), 3.06 (ddd, *J* = 19.4, 13.0, 7.3 Hz, 2H).

### 1.43. Synthesis of Final Compound 105

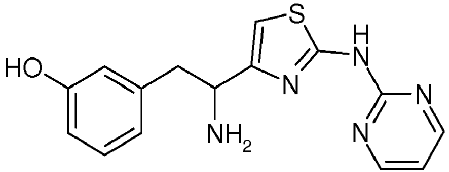

Compound **105** was synthesized following Method E by reaction of Intermediate LII (490 mg, 1 eq) and methylhydrazine (1 mL, 20 eq) in EtOH (20 mL). The compound was purified in silica gel (0% to 10% MeOH in DCM) to give Final Compound **105** (7 mg, white solid, 2%) as a side product of this synthesis.
HPLC-MS (method 1): Rt = 4.7 min [M+H]+ 297.1 (fragmentation).
1H NMR (300 MHz, DMSO) δ 9.23 (bs, 1H), 8.62 (d, *J* = 4.8 Hz, 2H), 7.02 (m, 2H), 6.78 (s, 1H), 6.57 (m, 1H), 6.55 (m, 2H), 4.08 (m, 1H), 3.05 (dd, *J* = 13.4, 5.9 Hz, 1H), 2.74 (m, 1H).

### 1.44. Synthesis of Final Compound 120

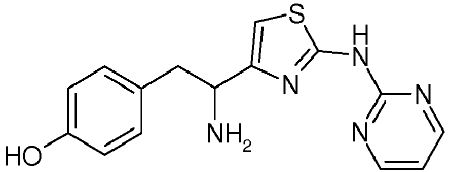

Compound **120** was synthesized following Method E by reaction of Intermediate CXXIII (75 mg, 1 eq) and hydrazine hydrate (0.1 mL, 6 eq) in EtOH (3 mL). The compound was purified in silica gel (0% to 20% MeOH in EtOAc) to give Final Compound **120** (24 mg, white solid, 48%).
HPLC-MS (method 1): Rt = 2.1 min [M+H]+ 297.1 (fragmentation).
1H NMR (300 MHz, DMSO) δ 9.21 (s, 1H), 8.62 (d, *J* = 4.8 Hz, 2H), 7.02 (t, *J* = 4.8 Hz, 1H), 6.90 (d, *J* = 8.5 Hz, 2H), 6.81 (s, 1H), 6.62 (d, *J* = 8.5 Hz, 2H), 4.13 (t, *J* = 6.8 Hz, 1H), 3.04 (dd, *J* = 13.4, 6.6 Hz, 1H), 2.81 (dd, *J* = 13.2, 6.9 Hz, 1H).

### 1.45. Synthesis of Final Compound 122

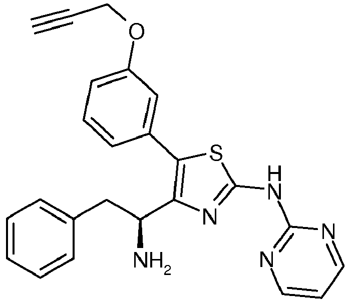

Compound **122** was purified from Final Compound **87** following Method F. Chiral column: Chiral IE; Heptane/EtOH/EDA 70:30:0.1; 6 mL/min; 40 min; 300 nm; Rt= 10.07 min. The product was isolated contaminated with salts of EDA and it was purified by chromatography in silica gel (5% to 10% MeOH in DCM) to render Final Compound 122 (4 mg, white solid, ee > 99%).
LCMS (method 1): Rt = 3.7 min; [M+H]+ 411.1 (fragmentation).
1H NMR (700 MHz, DMSO) δ 8.64 (d, *J* = 4.8 Hz, 2H), 7.27-7.22 (m, 1H), 7.16 (t, *J* = 7.3 Hz, 2H), 7.12 (t, *J* = 7.2 Hz, 1H), 7.04 (t, *J* = 4.8 Hz, 1H), 6.97 (d, *J* = 7.2 Hz, 2H), 6.92 (dd, *J* = 8.3, 1.8 Hz, 1H), 6.74 (s, 1H), 6.70 (t, *J* = 9.6 Hz, 1H), 4.76 (d, *J* = 2.2 Hz, 2H), 4.09 (dd, *J* = 17.0, 10.3 Hz, 1H), 3.57 (t, *J* = 2.2 Hz, 1H), 3.08 (dt, *J* = 27.8, 13.9 Hz, 1H), 3.02 - 2.93 (m, 1H).

### 1.46. Synthesis of Final Compound 123

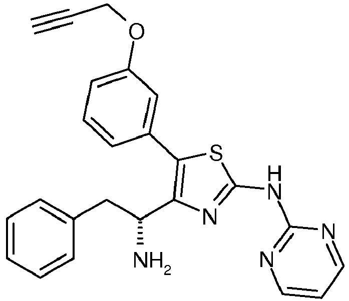

Compound **123** was purified from Final Compound **87** following Method F. Chiral column: Chiral IE; Heptane/EtOH/EDA 70:30:0.1; 6 mL/min; 40 min; 300 nm; Rt= 28.49 min. The product was isolated contaminated with salts of EDA and it was purified by chromatography in silica gel (5% to 10% MeOH in DCM) to render Final Compound **123** (3 mg, white solid, ee > 99%).
LCMS (Method 1): Rt = 3.7 min; [M+H]+ 411.1 (fragmentation).
1H NMR (700 MHz, DMSO) δ 8.63 (d, *J= 4.8* Hz, 2H), 7.27 - 7.22 (m, 1H), 7.16 (t, *J* = 7.3 Hz, 2H), 7.12 (t, *J* = 7.2 Hz, 1H), 7.03 (t, *J* = 4.8 Hz, 1H), 6.97 (d, *J* = 7.2 Hz, 2H), 6.92 (dd, *J* = 8.3, 1.8 Hz, 1H), 6.74 (s, 1H), 6.70 (t, *J* = 9.6 Hz, 1H), 4.76 (d, *J* = 2.2 Hz, 2H), 4.09 (dd, *J* = 17.0, 10.3 Hz, 1H), 3.57 (t, *J* = 2.2 Hz, 1H), 3.08 (dt, *J* = 27.8, 13.9 Hz, 1H), 3.01 - 2.94 (m, 1H).

### 1.47. Synthesis of Final Compound 124

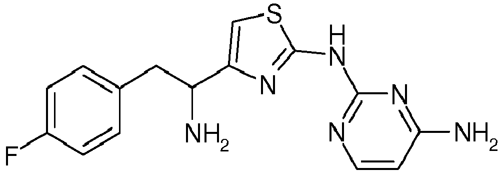

Compound **124** was prepared following similar synthetic strategy that the one used for the synthesis of Intermediate CLVI by reaction of Intermediate XXXVI with tert-butyl (2-chloropyrimidin-4-yl)carbamate and further BOC and phthalimide deprotection to yield Final Compound **124.**
LCMS (method 3): Rt = 2.7 min; [M+H]+ 331.1.
1H NMR (300 MHz, DMSO) δ 10.91 (br s, 1H), 7.92 (d, *J* = 5.8 Hz, 1H), 7.17 - 7.10 (m, 2H), 7.09 - 7.01 (m, 2H), 6.83 (s, 2H), 6.62 (d, *J* = 0.6 Hz, 1H), 6.02 (d, *J* = 5.8 Hz, 1H), 4.04 - 3.94 (m, 1H), 3.06 (dd, *J* = 13.3, 5.6 Hz, 1H), 2.80 (dd, *J* = 13.3, 7.5 Hz, 1H).

### - Example 2. TRF1 inhibition activity

### Measurement of TRF1 levels at telomere by immunofluorescence

Assay was performed in CHA9.3, a primary murine cell line derived from a lung cancer. (Garcia-Beccaria *et al*., 2015). Cells were grown in DMEM (Gibco) supplemented with 10% FBS (fetal bovine serum), 1% penicillin/estreptomicen (Someglas 100U/ml) and 0.5% Amphotericin B (Gibco). Cells were incubated at 37ºC with 5% CO₂. Cells were trypsinized with Trypsin-EDTA (Sigma) and washed with PBS (BioWhittaker) before counting and plating.

Cells were seeded in 10000 cells per 150 µl per well into black 96-well plates (Falcon) and incubated for 16 h at 37°C, 5% CO2. On day two, duplicates of each compound at 10 µM in DMSO were tested. The compounds were added to duplicate wells in 96-well cell plates using a FX BECKMAN robot (BeckmanCoulter) and were incubated at 37°C under CO₂ atmosphere. After 24 hours, relative levels of TRF1 at telomeres were quantified by immunofluorescence. A rat monoclonal antibody against mouse TRF1 developed by the Monoclonal antibody unit at CNIO was used to detect endogenous TRF1.

First cells were fixed with 4 % formaldehyde (Sigma) in PBS (Alaos) during 13 minutes at room temperature (RT). After removal of the medium, cells were washed twice with PBS 10 minutes. Cells were permeabilized with a solution 0.2%Triton X-100 (Sigma) in PBS during 10 minutes at RT. After two more washes with PBS, cells were incubated with a solution 0.1%NaCitrate (Fluka) /0.1 %Triton X100 during 5 minutes at RT. After, two more washes with PBS cells were blocked with a solution 3% BSA (Sigma) in PBS-T, PBS with 0.05% Tween20 (Sigma), during 3 o 4 hours. Then, cells were incubated o/n at 4ºC with a dilution 1:750 of anti TRF1 antibody. After two washes of 10 minutes with PBS-T, cells were incubated with secondary antibody anti-rat 555 (Alexa Fluor) (1 :500) during 1 hour at RT: Then, 3 washes with PBS-T (0.05%) of 10 minutes and a last wash with PBS1X were performed. Nuclear staining was performed with Hoechst (Invitrogen) at de 10µg/µL in PBS1X during10 minutes.

For quantification of TRF1 foci intensity, pictures of fixed cells were automatically acquired from each well by the Opera High Content Screening (HCS) system (Perkin Elmer). Forty images of random fields per well, with a 40xmagnification lens, were taken under non-saturating conditions. At least 1 × 10³ cells were analyzed for each well. Briefly, images were segmented using the DAPI staining to generate masks matching cell nuclei from which-TRF1 foci were analyzed. Automatic analysis was performed with Acapela v2.0 software. Quantification of the inhibition of TRF1 levels at telomeres was performed with Pipeline platform.

### TRF1 inhibition activity

Following the above technique, the percentage of inhibition of TRF1 levels was quantified for each one of the compounds whose synthesis is described in Example 1. The results of the percentage of TRF1 inhibition quantified for each compound are shown in Table 1 below.

**Table 1. Percentage of inhibition of TRF1 for each compound**

| **Compound No.** | **% inhibition of TRF1 levels** |
|---|---|
| 1 | 27.32 |
| 2 | 29.83 |
| 3 | 25.35 |
| 4 | 24.91 |
| 5 | 29.83 |
| 6 | 28.27 |
| 7 | 28.55 |
| 8 | 32.38 |
| 12 | 27.93 |
| 14 | 0 |
| 17 | 31.57 |
| 18 | 33.77 |
| 19 | 27.46 |
| 20 | 19.40 |
| 21 | 28.99 |
| 22 | 0 |
| 26 | 0 |
| 27 | 13.71 |
| 28 | 0 |
| 31 | 28.72 |
| 32 | 26.62 |
| 33 | 30.01 |
| 34 | 20.98 |
| 35 | 20.51 |
| 36 | 18.39 |
| 37 | 9.77 |
| 52 | 10.50 |
| 58 | 27.06 |
| 60 | 24.78 |
| 61 | 30.53 |
| 62 | 11.45 |
| 63 | 11.67 |
| 64 | 6.41 |
| 66 | 18.07 |
| 68 | 31.25 |
| 69 | 7.70 |
| 74 | 15.46 |
| 78 | 27.77 |
| 81 | 19.71 |
| 83 | 17.17 |
| 84 | 16.29 |
| 87 | 17.50 |
| 88 | 29.95 |
| 89 | 22.97 |
| 105 | 29.49 |
| 120 | 20.00 |
| 122 | 5.33 |
| 123 | 10.90 |
| 124 | 10.50 |
| 124 | 29.79 |

### References

García-Cao I, García-Cao M, Tomás-Loba A, Martín-Caballero J, Flores JM, Klatt P, Blasco MA, Serrano M. Increased p53 activity does not accelerate telomere-driven ageing. EMBO Rep. 2006 May; 7(5):546-52
Baerlocher, G. M., Oppliger Leibundgut, E., Ottmann, O. G., Spitzer, G., Odenike, O., McDevitt, M. a., Röth, A., Daskalakis, M., Burington, B., Stuart, M. and Snyder, D. S. (2015) 'Telomerase Inhibitor Imetelstat in Patients with Essential Thrombocythemia.', The New England journal of medicine, 373(10), pp. 920-8. doi: 10.1056/NEJMoa1503479
Bainbridge, M. N., Armstrong, G. N., Gramatges, M. M., Bertuch, A. A., Jhangiani, S. N., Doddapaneni, H., Lewis, L., Tombrello, J., Tsavachidis, S., Liu, Y., Jalali, A., Plon, S. E., Lau, C. C., Parsons, D. W., Claus, E. B., Barnholtz-Sloan, J., Il'yasova, D., Schildkraut, J., Ali-Osman, F., Sadetzki, S., Johansen, C., Houlston, R. S., Jenkins, R. B., Lachance, D., Olson, S. H., Bernstein, J. L., Merrell, R. T., Wrensch, M. R., Walsh, K. M., Davis, F. G., Lai, R., Shete, S., Aldape, K., Amos, C. I., Thompson, P. A., Muzny, D. M., Gibbs, R. A., Melin, B. S. and Bondy, M. L.
(2015) 'Germline mutations in shelterin complex genes are associated with familial glioma', J Natl Cancer Inst, 107(1), p. 384. doi: 10.1093/jnci/dju384 Bejarano L, Schuhmacher AJ, Mendez M, Megías D, Blanco-Aparicio C, Martinez S, Pastor J, Squatrito M, Blasco MA. Inhibition of TRF1 Telomere Protein Impair Tumor Initiation an Progression in Gliobastoma Mouse Models and Patient-Derived Xenografts. Cancer Cell 2017, 32:590-607
Boué S., Paramonov, I., Barrero, M. J. and Belmonte, J. C. I. (2010) 'Analysis of human and mouse reprogramming of somatic cells to induced pluripotent stem cells. what is in the plate?', PLoS ONE, 5(9), pp. 1-14. doi: 10.1371/journal.pone.0012664
Calvete O., Martinez, P., Garcia-Pavia, P., Benitez-Buelga, C., Paumard-Hernández, B., Fernandez, V., Dominguez, F., Salas, C., Romero-Laorden, N., Garcia-Donas, J., Carrillo, J., Perona, R., Triviño, J. C., Andrés, R., Cano, J. M., Rivera, B., Alonso-Pulpon, L., Setien, F., Esteller, M., Rodriguez-Perales, S., Bougeard, G., Frebourg, T., Urioste, M., Blasco, M. A. and Benítez, J. (2015) 'A mutation in the POT1 gene is responsible for cardiac angiosarcoma in TP53-negative Li-Fraumeni-like families.', Nature communications, 6, p. 8383. doi: 10.1038/ncomms9383
Chin, L., Artandi, S. E., Shen, Q., Tam, A., Lee, S. L., Gottlieb, G. J., Greider, C. W. and DePinho, R. A. (1999) 'p53 deficiency rescues the adverse effects of telomere loss and cooperates with telomere dysfunction to accelerate carcinogenesis', Cell, 97(4), pp. 527-538. doi: 10.1016/S0092-8674(00)80762-X.
Daniel El Fassi, Bjørn, M., Nielsen, C. and Hasselbalch, H. (2015) 'Telomerase Inhibitor Imetelstat in Essential Thrombocythemia and Myelofibrosis', New England Journal of Medicine, 373(26), pp. 2579-2581. doi: 10.1056/NEJMc1512663
De Lange, T. (2002) 'Protection of mammalian telomeres', Oncogene, pp. 532-540. doi: 10.1038/sj/onc/1205080.
De Lange, T. (2005) 'Shelterin: The protein complex that shapes and safeguards human telomeres', Genes and Development, pp. 2100-2110. doi: 10.1101/gad.1346005
Garcia-Beccaria M., Martinez P., Méndez-Pertuz M., Martinez S., Blanco-Aparicio C., Cañamero M., Mulero F., Ambrogio C., Flores J.M., Megias D., et al., Therapeutic inhibition of TRF1 impairs the growth of p53-deficient K-RasG12V-induced lung cancer by induction of telomeric DNA damage. EMBO Mol. Med. 2015; 7: 930-949
Gonzalez-Suarez, E., Samper, E., Flores, J. M. and Blasco, M. A. (2000) 'Telomerase-deficient mice with short telomeres are resistant to skin tumorigenesis.', Nature genetics, 26(1), pp. 114-117. doi: 10.1038/79089
Greenberg, R. A., Chin, L., Femino, A., Kee-Ho, L., Gottlieb, G. J., Singer, R. H., Greider, C. W. and DePinho, R. A. (1999) 'Short dysfunctional telomeres impair tumorigenesis in the INK4a(??2/3) cancer-prone mouse', Cell, 97(4), pp. 515-525. doi: 10.1016/S0092-8674(00)80761-8
Houghtaling, B. R., Cuttonaro, L., Chang, W. and Smith, S. (2004) 'A dynamic molecular link between the telomere length regulator TRF1 and the chromosome end protector TRF2', Current Biology, 14(18), pp. 1621-1631. doi: 10.1016/j.cub.2004.08.052
Joseph, I., Tressler, R., Bassett, E., Harley, C., Buseman, C. M., Pattamatta, P., Wright, W. E., Shay, J. W. and Go, N. F. (2010) 'The telomerase inhibitor imetelstat depletes cancer stem cells in breast and pancreatic cancer cell lines', Cancer Research, 70(22), pp. 9494-9504. doi: 10.1158/0008-5472.CAN-10-0233
Kim, N., Piatyszek, M., Prowse, K., Harley, C., West, M., Ho, P., Coviello, G., Wright, W., Weinrich, S. and Shay, J. (1994) 'Specific association of human telomerase activity with immortal cells and cancer', Science, 266(5193), pp. 2011-2015. doi: 10.1126/science. 7605428
Liu D., O'Connor, M. S., Qin, J. and Songyang, Z. (2004) 'Telosome, a mammalian telomere-associated complex formed by multiple telomeric proteins', Journal of Biological Chemistry, 279(49), pp. 51338-51342. doi: 10.1074/jbc.M409293200
Martinez, P., Thanasoula, M., Muñoz, P., Liao, C., Tejera, A., McNees, C., Flores, J. M., Fernandez-Capetillo, O., Tarsounas, M. and Blasco, M. A. (2009) 'Increased telomere fragility and fusions resulting from TRF1 deficiency lead to degenerative pathologies and increased cancer in mice', Genes and Development, 23(17), pp. 2060-2075. doi: 10.1101/gad.543509
Middleton, G., Silcocks, P., Cox, T., Valle, J., Wadsley, J., Propper, D., Coxon, F., Ross, P., Madhusudan, S., Roques, T., Cunningham, D., Falk, S., Wadd, N., Harrison, M., Corrie, P., Iveson, T., Robinson, A., McAdam, K., Eatock, M., Evans, J., Archer, C., Hickish, T., Garcia-Alonso, A., Nicolson, M., Steward, W., Anthoney, A., Greenhalf, W., Shaw, V., Costello, E., Naisbitt, D., Rawcliffe, C., Nanson, G. and Neoptolemos, J. (2014) 'Gemcitabine and capecitabine with or without telomerase peptide vaccine GV1001 in patients with locally advanced or metastatic pancreatic cancer (TeloVac): An open-label, randomised, phase 3 trial', The Lancet Oncology, 15(8), pp. 829-840. doi: 10.1016/S1470-2045(14)70236-0
Nwe K. and Brechbiel MW. Growing Applications of "Click Chemistry" for Bioconjugation in Contemporary Biomedical Research. Cancer Biother Radiopharm 2009, 24(3): 289-302.
Parkhurst, M. R., Riley, J. P., Igarashi, T., Li, Y., Robbins, P. F. and Rosenberg, S. A. (2004) 'Immunization of patients with the hTERT:540-548 peptide induces peptide-reactive T lymphocytes that do not recognize tumors endogenously expressing telomerase', Clinical Cancer Research, 10(14), pp. 4688-4698. doi: 10.1158/1078-0432.CCR-04-0325
Perera, S. A., Maser, R. S., Xia, H., McNamara, K., Protopopov, A., Chen, L., F.hezel, A., Kim, C. F., Bronson, R. T., Castrillon, D. H., Chin, L., Bardeesy, N., DePinho, R. A. and Wong, K. K. (2008) 'Telomere dysfunction promotes genome instability and metastatic potential in a K-ras p53 mouse model of lung cancer', Carcinogenesis, 29(4), pp. 747-753. doi: 10.1093/carcin/bgn050
Ramsay A. J., Quesada, V., Foronda, M., Conde, L., Martínez-Trillos, A., Villamor, N., Rodriguez, D., Kwarciak, A., Garabaya, C., Gallardo, M., López-Guerra, M., López-Guillermo, A., Puente, X. S., Blasco, M. A., Campo, E. and López-Otín, C. (2013) 'POT1 mutations cause telomere dysfunction in chronic lymphocytic leukemia', Nature genetics, 45(5), pp. 526-530. doi: 10.1038/ng.2584
Robles- Espinoza, C. D., Harland, M., Ramsay, A. J., Aoude, L. G., Quesada, V., Ding, Z., Pooley, K. A., Pritchard, A. L., Tiffen, J. C., Petljak, M., Palmer, J. M., Symmons, J., Johansson, P., Stark, M. S., Gartside, M. G., Snowden, H., Montgomery, G. W., Martin, N. G., Liu, J. Z., Choi, J., Makowski, M., Brown, K. M., Dunning, A. M., Keane, T. M., Lopez-Otin, C., Gruis, N. A., Hayward, N. K., Bishop, D. T., Newton-Bishop, J. A. and Adams, D. J. (2014) 'POT1 loss-of-function variants predispose to familial melanoma', Nat Genet, 46(5), pp. 478-481. doi: 10.1038/ng.2947
Schneider R. P., Garrobo, I., Foronda, M., Palacios, J. a, Marion, R. M., Flores, I., Ortega, S. and Blasco, M. a (2013) 'TRF1 is a stem cell marker and is essential for the generation of induced pluripotent stem cells.', Nature communications, 4, p. 1946. doi: 10.1038/ncomms2946
Shay, J. W. and Bacchetti, S. (1997) 'A survey of telomerase activity in human cancer.', European journal of cancer (Oxford, England: 1990), 33(5), pp. 787-91. doi: 10.1016/S0959-8049(97)00062-2
Shi, J., Yang, X. R., Ballew, B., Rotunno, M., Calista, D., Fargnoli, M. C., Ghiorzo, P., Bressac-de Paillerets, B., Nagore, E., Avril, M. F., Caporaso, N. E., McMaster, M. L., Cullen, M., Wang, Z., Zhang, X., Group, N. D. C. S. W., Laboratory, N. D. C. G. R., French Familial Melanoma Study, G., Bruno, W., Pastorino, L., Queirolo, P., Banuls-Roca, J., Garcia-Casado, Z., Vaysse, A., Mohamdi, H., Riazalhosseini, Y., Foglio, M., Jouenne, F., Hua, X., Hyland, P. L., Yin, J., Vallabhaneni, H., Chai, W., Minghetti, P., Pellegrini, C., Ravichandran, S., Eggermont, A., Lathrop, M., Peris, K., Scarra, G. B., Landi, G., Savage, S. A., Sampson, J. N., He, J., Yeager, M., Goldin, L. R., Demenais, F., Chanock, S. J., Tucker, M. A., Goldstein, A. M., Liu, Y. and Landi, M. T. (2014) 'Rare missense variants in POT1 predispose to familial cutaneous malignant melanoma', Nat Genet, 46(5), pp. 482-486. doi: 10.1038/ng.2941
Singh MS, Chowdhury S, Koley S. Advances of azide-alkyne cycloaddition-click chemistry over the recent decade. Tetrahedron 2016, 72: 5257 - 5283.
Tefferi A., Lasho, T. L., Begna, K. H., Patnaik, M. M., Zblewski, D. L., Finke, C. M., Laborde, R. R., Wassie, E., Schimek, L., Hanson, C. a., Gangat, N., Wang, X. and Pardanani, A. (2015) 'A Pilot Study of the Telomerase Inhibitor Imetelstat for Myelofibrosis.', New England Journal of Medicine, 373(10), pp. 908-919. doi: 10.1056/NEJMoa1310523
Zhang J., Jima, D., Moffitt, A. B., Liu, Q., Czader, M., Hsi, E. D., Fedoriw, Y., Dunphy, C. H., Richards, K. L., Gill, J. I., Sun, Z., Love, C., Scotland, P., Lock, E., Levy, S., Hsu, D. S., Dunson, D. and Dave, S. S. (2014) 'The genomic landscape of mantle cell lymphoma is related to the epigenetically determined chromatin state of normal B cells', Blood, 123(19), pp. 2988-2996. doi: 10.1182/blood-2013-07-517177

## Claims

1. A compound of Formula I, 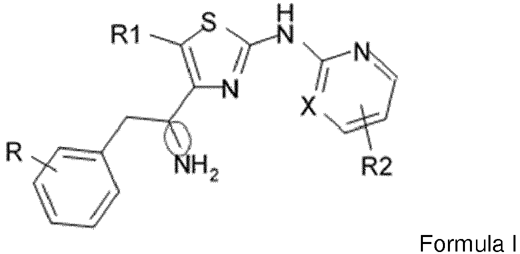 wherein,
R is selected from H, halo, alkyl, O-alkyl, N-alkyl, alkinyl, O-alkinyl,
R1 is selected from H, halo, aryl (unsubstituted or substituted),
X is C or N,
R2 is selected from H, halo, alkyl, O-alkyl, amino, alkenyl, or combinations thereof,
and
the bond surrounded by an oval can represent the racemate, the R-enantiomer or the S-enantiomer with regard to the -NH2 group linked by said bond to the structure, provided that
when X is N, R, R1 and R2 are not simultaneously H.

2. The compound according to claim 1, wherein
R is selected from H, halo (fluoro, chloro, bromo) or O-alkinyl,
R1 is selected from H, bromo, or substituted phenyl (Ph),
X is C or N,
and/or
R2 is selected from H, bromo, CH₃, OCH₃, CCH, and the combination of CH₃, and OCH3 each one being in one of the meta positions.

3. The compound according to claim 1 or 2, which is selected of the group of compounds wherein
the compound is a racemate with regard to the amino bond; R is ortho-Br; R1 is H; X is N; R2 is H
the compound is the R-enantiomer with regard to the amino bond; R is ortho-Br; R1 is H; X is N; R2 is H
the compound is the S-enantiomer with regard to the amino bond; R is ortho-Br; R1 is H; X is N; R2 is H,
the compound Is a racemate with regard to the amino bond; R is meta-Br; R1 is H; X is N; R2 is H,
the compound is the R-enantiomer with regard to the amino bond; R is meta-Br; R1 is H; X is N; R2 is H,
the compound is the S-enantiomer with regard to the amino bond; R is meta-Br; R1 is H; X is N; R2 is H,
the compound Is a racemate with regard to the amino bond; R is para-Br; R1 is ; X is N; R2 is H,
the compound Is a racemate with regard to the amino bond; R is meta-CI; R1 is H; X is N; R2 is H,
the compound Is a racemate with regard to the amino bond; R is meta-OCH2CCH; R1 is H; X is N; R2 is H,
the compound is the R-enantiomer with regard to the amino bond; R is meta-OCH₂CCH; R1 is H; X is N; R2 is H,
the compound is the S-enantiomer with regard to the amino bond; R is meta-OCH₂CCH; R1 is H; X is N; R2 is H,
the compound Is a racemate with regard to the amino bond; R is para-OCH2CCH; R1 is H; X is N; R2 is H,
the compound is the R-enantiomer with regard to the amino bond; R is para-OCH₂CCH; R1 is H; X is N; R2 is H,
the compound is the R-enantiomer with regard to the amino bond; R is para-O(CH₂)₄CCH; R1 is H; X is N; R2 is H,
the compound is the S-enantiomer with regard to the amino bond; H; R1 is H; X is N; R2 is H,
the compound is the S-enantiomer with regard to the amino bond; H; R1 is H; X is N; R2 is 5-Br,
the compound is the S-enantiomer with regard to the amino bond; H; R1 is H; X is N; R2 is H,
the compound is the S-enantiomer with regard to the amino bond; H; R1 is H; X is N; R2 is 5-CH₃,
the compound is the S-enantiomer with regard to the amino bond; H; R1 is H; X is N; R2 is 4-CH₃ and 6-OCH₃,
the compound is a racemate with regard to the amino bond; R is para-NH2; R1 is H; X is N; R2 is H,
the compound Is a racemate with regard to the amino bond; R is meta-CCH; R1 is H; X is N; R2 is H,
the compound is the R-enantiomer with regard to the amino bond; R is meta-CCH; R1 is H; X is N; R2 is H,
the compound is the S-enantiomer with regard to the amino bond; R is meta-CCH; R1 is H; X is N; R2 is H,
the compound Is a racemate with regard to the amino bond; H; R1 is H; X is N; R2 is 5-CCH,
the compound is the R-enantiomer with regard to the amino bond; H; R1 is H; X is N; R2 is 5-CCH,
the compound is the S-enantiomer with regard to the amino bond; H; R1 is H; X is N; R2 is 5-CCH,
the compound Is a racemate with regard to the amino bond; R is para-CCH; R1 is H; X is N; R2 is H,
the compound is the R-enantiomer with regard to the amino bond; R is para-CCH; R1 is H; X is N; R2 is H,
the compound is the S-enantiomer with regard to the amino bond; R is para-CCH; R1 is H; X is N; R2 is H,
the compound Is a racemate with regard to the amino bond; R is para-F; R1 is H; X is N; R2 is 5-NHCO(CH₂)₂CCH,
the compound Is a racemate with regard to the amino bond; R is ortho-Br; R1 is meta-PhNH₂; X is N; R2 is H,
the compound Is a racemate with regard to the amino bond; R is H; R1 is meta-PhCCH; X is N; R2 is H,
the compound Is a racemate with regard to the amino bond; R is H; R1 is meta-Ph-NHCOCH₃; X is N; R2 is H,
the compound is the S-enantiomer with regard to the amino bond; R is H; R1 is meta-Ph-O-CH₂CH₂OCH₃; X is N; R2 is H,
the compound Is a racemate with regard to the amino bond; R is H; R1 is meta-Ph-O-CH₂CCH; X is N; R2 is H,
the compound is the S-enantiomer with regard to the amino bond; R is H; R1 is Br; X is N; R2 is H,
the compound is the S-enantiomer with regard to the amino bond; R is H; R1 is meta-PhOH; X is N; R2 is H,
the compound Is a racemate with regard to the amino bond; R is meta-OH; R1 is H; X is N; R2 is H,
the compound is a racemate with regard to the amino bond; R is para-OH; R1 is H; X is N; R2 is H,
the compound is the S-enantiomer with regard to the amino bond; R is H; R1 is meta-PhOCH₂CCH; X is N; R2 is H,
the compound is the R-enantiomer with regard to the amino bond; R is H; R1 is meta-PhOCH₂CCH; X is N; R2 is H, and
the compound Is a racemate with regard to the amino bond; R is para-F; R1 is H; X is N; R2 is 4-NH₂.

4. The compound according to claim 1, wherein R, R1 or R2 presents a CCH moiety at one end.

5. The compound according to claim 4, wherein the compound is selected from the group of compounds wherein,
R is meta-O-CH₂CCH, para- O-CH₂CCH, para-O(CH₂)₄CCH, meta-CCH or para-CCH,
R1 is PhOCH₂CCH, o, PhCCH, or
R2 is 5-CCH, 5-NHCO(CH₂)₂CCH

6. A composition which comprises a compound of claim 2 or 3 and a pharmaceutically acceptable excipient, diluent or carrier.

7. A composition according to claim 6, which additionally comprises another antitumoral compound.

8. A composition according to claim 7, which additionally comprises at least an additional TRF1 inhibitor.

9. A composition according to claim 8, wherein the additional TRF1 inhibitor is selected from the group of:
a. a compound which acts through the Akt/PI3K pathway;
b. the compound of Formula I where X is N and R, R1 and R2 are H;
c. a compound selected from the group of: an RTK inhibitor, a MEK inhibitor, an ERK inhibitor, an HSP90 inhibitor, docetaxel and gemcitabine.

10. A composition according to claim 8 or 9, which additionally comprises temozolomide.

11. A compound of claim 2 or 3 or a composition of any one of claims 6 to 10 for use as a medicament.

12. A compound of claim 2 or 3 or a composition of any one of claims 6 to 10, for use in the prevention or treatment or cancer.

13. A compound of claim 2 or 3 or a composition of any one of claims 6 to 10, for use according to claim 12, wherein the use is in the prevention of a cancer **characterized by** high content of cancer stem cells.

14. A compound of claim 2 or 3 or a composition of any one of claims 6 to 10 for use according to claim 12 or 13, wherein the use is in the prevention of recurrence of treatment of glioblastoma multiforme or lung carcinoma.

15. Use of a compound of any one of claims 1 to 3 or any one of claims 4 to 5 as a research tool in the development of TRF1 inhibitors and/or the research about telomeres and/or TRF1 mechanism of action.

16. Use of a compound of any one of claims 4 and 5 as precursor to make a chemical proof for cell imaging studies, cellular localization studies and/or pull-down assays.
